# EUROPEAN PATENT APPLICATION

(11) **EP 4 851 191 A1**
(43) Date of publication of application: **09.12.2026**
(21) Application number: 25747731.5
(22) Date of filing: 23.01.2025
(51) Int. Cl.: C07K 16/28, C12N 15/13, A61K 39/395, A61K 47/68, A61K 51/10, A61P 35/00, A61P 37/06

(54) **MONOCLONAL ANTIBODY SPECIFICALLY BINDING TO PD-1 AND PHARMACEUTICAL USE THEREOF**

(30) Priority: 02.02.2024 CN 202410159528
(71) Applicant: INNOLAKE BIOPHARMA (HANGZHOU) CO., LTD., Shanghai 200126 (CN)
(72) Inventor: QIU, Junzhuan, Shanghai 200126 (CN); WANG, Zhensheng, Shanghai 200126 (CN); SUN, Kai, Shanghai 200126 (CN); SUN, Jian, Shanghai 200126 (CN); CHEN, Junyong, Shanghai 200126 (CN); LI, Zhongliang, Shanghai 200126 (CN); XIA, Mingde, Shanghai 200126 (CN); CHEN, Rulei, Shanghai 200126 (CN)
(74) Representative: Epping - Hermann - Fischer
(86) International application number: PCT/CN2025/074146
(87) International publication number: WO 2025/162161

(57) **Abstract**

The present invention relates to the field of immunotherapy and molecular immunology, and in particular relates to a monoclonal antibody that specifically binds PD-1 or an antigen-binding fragment thereof, as well as pharmaceutical uses thereof. The antibody or antigen-binding fragment thereof can be particularly used for preventing or treating cancers, inflammation and autoimmune diseases.

## Description

### TECHNICAL FIELD

The present invention relates to the field of immunotherapy and molecular immunology, and in particular relates to a monoclonal antibody that specifically binds PD-1 or an antigen-binding fragment thereof, as well as pharmaceutical uses thereof. The antibody or antigen-binding fragment thereof can be particularly used for preventing or treating cancers, inflammation and autoimmune diseases.

### BACKGROUND ART

Programmed Death Receptor 1 (PD-1), also known as CD279, is encoded by the PDCD1 gene located at region 37 band 3 in the long arm of chromosome 2 (2q37.3) and is a 55 kDa transmembrane protein (Agata, et al., (1996), Int. Immunol. 8:765-772). PD-1 is a member of the immunoglobulin B7-CD28 family, consisting of an extracellular region, a hydrophobic transmembrane region and an intracellular region. The intracellular segment contains an immunoreceptor tyrosine-based inhibitory motif (ITIM) and an immunoreceptor tyrosine-based switch motif (ITSM). Activation of ITSM is closely correlated with effector T cell activity (Ishida, et al., (1992) EMBO J 11: 3887-3895). PD-1 protein is expressed on B lymphocytes, T lymphocytes, natural killer (NK) cells, monocytes and dendritic cells (DCs) (Duraiswamy, et al., (2013) Cancer Res. 73:6900-12).

PD-1 has two ligands, namely PD-L1 (also known as B7-H1, CD274) and PD-L2 (also known as B7-DC, CD273), both of which are membrane proteins with IgV-like and IgC-like domains in the extracellular region. PD-L1 is widely expressed on the surfaces of hematopoietic cells, non-hematopoietic cells and tumor cells (Lesterhuis, et al., (2011) Mol Immunol 49:1-3). PD-L1 is considered the primary ligand of PD-1, while PD-L2 exhibits 2-6 times higher affinity for PD-1 than PD-L1. PD-L2 is mainly expressed on antigen-presenting cells and Th2 cells, and is generally lowly expressed in other cell types; and therefore it generally has no influence on the binding between PD-L1 and PD-1 (Rozali, et al., (2012) Clin. Dev. Immunol. 2012:656340).

Activation of the PD-1/PD-L1 signaling pathway may induce immunosuppressive effects. It has been found that PD-L1-induced aggregation of PD-1 promotes the recruitment of SHP2 phosphatase, which further suppresses T cell activity by the dephosphorylation of CD28 (Hui et al., Science, 355:1428-1433 (2017)). On the contrary, deficiency or insufficient activity of PD-1 may lead to autoimmunity. For example, PD-1 knockout in C57BL/6 mice results in the development of lupus-like syndrome (see Nishimura et al., Immunity, 11:141-151 (1999)). In humans, mutations of the PD-1 gene are highly correlated with the onset and progression of systemic lupus erythematosus, type 1 diabetes mellitus, rheumatoid arthritis and multiple sclerosis (see Nielsen et al., Tissue Antigens, 62(6):492-497 (2003); Bertsias et al., Arthritis & Rheumatism, 60(1): 207-218 (2009); Ni et al., Hum Genet 121(2) :223-232 (2007); Tahoori et al., Clin. Exp. Rheumatol, 29(5): 763-767 (2011) and Kroner et al., Ann.Neurol, 58(1):50-57). In this context, the development of a PD-1 agonist, such as a PD-1 agonist antibody, to promote PD-1 immunosuppressive signaling could potentially suppress or treat certain inflammatory or autoimmune diseases. This presents a new opportunity for the development of innovative antibody drugs targeting immune checkpoints and offers new insights into the treatment of some immune diseases.

### SUMMARY

The present inventors obtained anti-PD-1 agonistic monoclonal antibodies with excellent efficacy in boosting PD-1 activity via hybridoma technology, and successfully performed humanization on such antibodies. Such antibodies have broad prospects in the preparation of medicaments for activating PD-1 activity or level, as well as for the treatment or adjuvant treatment of inflammation and autoimmune diseases.

In one aspect, the present disclosure provides a monoclonal antibody or antigen-binding fragment thereof that specifically binds PD-1, comprising heavy chain CDRs and light chain CDRs, wherein:
the amino acid sequence of heavy chain CDR1 comprises SEQ ID NOs: 15, 21, 27, 33, 39, 50 or 56, or a sequence having two or fewer substituted amino acids relative to SEQ ID NOs: 15, 21, 27, 33, 39, 50 or 56;
the amino acid sequence of heavy chain CDR2 comprises SEQ ID NOs: 16, 22, 28, 34, 40, 51 or 57, or a sequence having two or fewer substituted amino acids relative to SEQ ID NOs: 16, 22, 28, 34, 40, 51 or 57, wherein X in SEQ ID NO: 40 is any amino acid;
the amino acid sequence of heavy chain CDR3 comprises SEQ ID NOs: 17, 23, 29, 35, 44, 52 or 58, or a sequence having two or fewer substituted amino acids relative to SEQ ID NOs: 17, 23, 29, 35, 44, 52 or 58;
the amino acid sequence of light chain CDR1 comprises SEQ ID NOs: 18, 24, 30, 36, 45, 53 or 59, or a sequence having two or fewer substituted amino acids relative to SEQ ID NOs: 18, 24, 30, 36, 45, 53 or 59;
the amino acid sequence of light chain CDR2 comprises SEQ ID NOs: 19, 25, 31, 37, 46, 54 or 60, or a sequence having two or fewer substituted amino acids relative to SEQ ID NOs: 19, 25, 31, 37, 46, 54 or 60; and
the amino acid sequence of light chain CDR3 comprises SEQ ID NOs: 20, 26, 32, 38, 47, 55 or 61, or a sequence having two or fewer substituted amino acids relative to SEQ ID NOs: 20, 26, 32, 38, 47, 55 or 61, wherein X in SEQ ID NO: 47 is any amino acid.

In some embodiments, X in the amino acid sequence of SEQ ID NO: 40 is N, Q or V.

In some embodiments, X in the amino acid sequence of SEQ ID NO: 47 is N or T.

In some embodiments, the substituted amino acids are conservatively substituted.

In some embodiments, the amino acid sequences of heavy chain CDR1, heavy chain CDR2, heavy chain CDR3, light chain CDR1, light chain CDR2 and light chain CDR3 are selected from one of the CDR combinations as shown in the following a to g:
a: the heavy chain CDR1, CDR2 and CDR3 sequences comprise SEQ ID NOs: 15-17 respectively; the light chain CDR1, CDR2 and CDR3 sequences comprise SEQ ID NOs: 18-20 respectively;
b: the heavy chain CDR1, CDR2 and CDR3 sequences comprise SEQ ID NOs: 21-23 respectively; the light chain CDR1, CDR2 and CDR3 sequences comprise SEQ ID NOs: 24-26 respectively;
c: the heavy chain CDR1, CDR2 and CDR3 sequences comprise SEQ ID NOs: 27-29 respectively; the light chain CDR1, CDR2 and CDR3 sequences comprise SEQ ID NOs: 30-32 respectively;
d: the heavy chain CDR1, CDR2 and CDR3 sequences comprise SEQ ID NOs: 33-35 respectively; the light chain CDR1, CDR2 and CDR3 sequences comprise SEQ ID NOs: 36-38 respectively;
e: the heavy chain CDR1, CDR2 and CDR3 sequences comprise SEQ ID NOs: 39, 40, 44 respectively; the light chain CDR1, CDR2 and CDR3 sequences comprise SEQ ID NOs: 45, 46, 47 respectively;
f: the heavy chain CDR1, CDR2 and CDR3 sequences comprise SEQ ID NOs: 50-52 respectively; the light chain CDR1, CDR2 and CDR3 sequences comprise SEQ ID NOs: 53-55 respectively; or
g: the heavy chain CDR1, CDR2 and CDR3 sequences comprise SEQ ID NOs: 56-58 respectively; the light chain CDR1, CDR2 and CDR3 sequences comprise SEQ ID NOs: 59-61 respectively.

In some embodiments, the antibody or antigen-binding fragment thereof comprises a heavy chain variable region and a light chain variable region, wherein the amino acid sequence of the heavy chain variable region is selected from any one of SEQ ID NOs: 1, 3, 5, 7, 9, 11 and 13; and the amino acid sequence of the light chain variable region is selected from any one of SEQ ID NOs: 2, 4, 6, 8, 10, 12 and 14.

In some embodiments, the antibody or antigen-binding fragment thereof comprises a heavy chain variable region and a light chain variable region, wherein the heavy chain variable region and the light chain variable region are humanized.

In some embodiments, the amino acid sequence of the humanized heavy chain variable region is selected from any one of SEQ ID NOs: 62, 64, 66, 68, 70, 71, 72 and 75; and the amino acid sequence of the humanized light chain variable region is selected from any one of SEQ ID NOs: 63, 65, 67, 69, 73, 74 and 76.

In some embodiments, the antibody or antigen-binding fragment thereof comprises a heavy chain and a light chain, wherein:
the amino acid sequence of the heavy chain is SEQ ID NO: 77, and the amino acid sequence of the light chain is selected from SEQ ID NO: 80 or SEQ ID NO: 81; or
the amino acid sequence of the heavy chain is SEQ ID NO: 78 or SEQ ID NO: 79, and the amino acid sequence of the light chain is SEQ ID NO: 81; or
the amino acid sequence of the heavy chain is SEQ ID NO: 82, and the amino acid sequence of the light chain is SEQ ID NO: 83; or
the amino acid sequence of the heavy chain is SEQ ID NO: 84, and the amino acid sequence of the light chain is SEQ ID NO: 85; or
the amino acid sequence of the heavy chain is SEQ ID NO: 86, and the amino acid sequence of the light chain is SEQ ID NO: 87; or
the amino acid sequence of the heavy chain is SEQ ID NO: 88, and the amino acid sequence of the light chain is SEQ ID NO: 89; or
the amino acid sequence of the heavy chain is SEQ ID NO: 90, and the amino acid sequence of the light chain is SEQ ID NO: 91.

In one aspect, the present disclosure provides a pharmaceutical composition comprising a therapeutically effective amount of the above antibody or antigen-binding fragment thereof and a pharmaceutically acceptable carrier.

In one aspect, the present disclosure provides use of the above antibody or antigen-binding fragment thereof in the manufacture of a medicament for activating PD-1 activity or PD-1 expression level, enhancing immune suppression by PD-1 in a subject, inhibiting T lymphocytes, inhibiting the expression of cytokines such as IFN-γ by T lymphocytes, and for the prevention, treatment or adjuvant treatment of inflammation and autoimmune diseases.

In one aspect, the present disclosure provides use of the above antibody or antigen-binding fragment thereof in the manufacture of a medicament for treating diseases or disorders, wherein the disease or disorder is cancer or autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In one aspect, the present disclosure provides a nucleic acid molecule encoding the above antibody or antigen-binding fragment thereof.

In one aspect, the present disclosure provides an expression vector comprising the sequence of the above nucleic acid molecule and expression regulatory sequences associated with the sequence.

In one aspect, the present disclosure provides a monoclonal antibody conjugate comprising a monoclonal antibody and a conjugate moiety, wherein the monoclonal antibody is the above antibody or antigen-binding fragment thereof, and the conjugate moiety is selected from one or more of radionuclides, agents, toxins, cytokines, cytokine receptor fragments, enzymes, fluorophores and biotin.

In one aspect, the present disclosure provides use of the above monoclonal antibody conjugate in the manufacture of a medicament for activating PD-1 activity or PD-1 expression level, enhancing immune suppression by PD-1 in a subject, inhibiting T lymphocytes, inhibiting the expression of cytokines such as IFN-γ by T lymphocytes, and for the prevention, treatment or adjuvant treatment of inflammation and autoimmune diseases.

In one aspect, the present disclosure provides a method for activating PD-1 activity or PD-1 expression level, enhancing immune suppression by PD-1 in a subject, inhibiting T lymphocytes, inhibiting the expression of cytokines such as IFN-γ by T lymphocytes, and for the prevention, treatment or adjuvant treatment of inflammation and autoimmune diseases, the method comprising administering a therapeutically effective amount of the above antibody or antigen-binding fragment thereof, or a therapeutically effective amount of the monoclonal antibody conjugate to a subject in need thereof.

In one aspect, the present disclosure provides a method for treating diseases or disorders in a subject in need thereof, the method comprising administering a therapeutically effective amount of the above antibody or antigen-binding fragment thereof, or a therapeutically effective amount of the monoclonal antibody conjugate to the subject, wherein the disease or disorder is cancer or autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In one aspect, the present disclosure provides a kit comprising:
a) the above antibody or the above pharmaceutical composition; and
b) instructions for use.

### BRIEF DESCRIPTION OF THE DRAWINGS

Figure 1: ELISA analysis of binding between PD-1 agonistic hybridoma antibodies and human PD-1 antigen protein.
Figure 2: ELISA analysis of binding between PD-1 agonistic hybridoma antibodies and cynomolgus PD-1 antigen protein.
Figure 3: Determination of activating activity of hybridoma antibodies against PD-1 via CD3-mediated reporter assay.
Figure 4: Determination of blocking activity of PD-1 agonistic hybridoma antibodies against PD-L1 binding to PD-1 via ELISA assay.
Figure 5: ELISA analysis of binding between humanized PD-1 agonistic antibodies and human PD-1.
Figure 6: ELISA analysis of binding between humanized PD-1 agonistic antibodies and cynomolgus PD-1.
Figure 7: Determination of activating activity of humanized PD-1 agonist antibodies against PD-1 molecule via CD3-induced reporter assay.
Figure 8: Determination of ADCC activity of humanized PD-1 agonist antibodies via ADCC reporter assay.
Figure 9: Determination of effect of humanized PD-1 agonist antibodies on T cell cytokine release via MLR assay.
Figure 10: Determination of effect of humanized PD-1 agonist antibodies on SEB-induced total CD4⁺ T cell proliferation.
Figure 11: Determination of effect of humanized PD-1 agonist antibodies on CEFTA-induced CD4⁺ memory T cell proliferation.
Figure 12: Determination of ADCC activity of humanized PD-1 agonist antibodies via ADCC reporter assay.
Figure 13: ELISA analysis of binding between affinity-matured PD-1 agonist antibodies and human PD-1.
Figure 14: FACS analysis of binding between affinity-matured PD-1 agonist antibodies and Jurkat-PD-1 cells.
Figure 15: Determination of activating effect of affinity-matured PD-1 agonist antibodies on PD-1 molecule via CD3 antibody-induced reporter assay.
Figure 16: Determination of cell killing activity of affinity-matured PD-1 agonist antibodies via FACS assay.
Figure 17: Determination of effect of affinity-matured PD-1 agonist antibodies on T cell cytokine release via MLR assay.
Figure 18: Determination of effect of affinity-matured PD-1 agonist antibodies on SEB-induced total CD4⁺ T cell proliferation.
Figure 19: Determination of effect of affinity-matured PD-1 agonist antibodies on CEFTA-induced CD4⁺ memory T cell proliferation.

### DETAILED DESCRIPTION

The present inventors obtained anti-PD-1 agonistic monoclonal antibodies with excellent efficacy in boosting PD-1 activity via hybridoma technology, and successfully performed humanization on such antibodies. Such antibodies have broad prospects in the preparation of medicaments for activating PD-1 activity or level, as well as for the treatment or adjuvant treatment of inflammation and autoimmune diseases.

The present disclosure provides monoclonal antibodies specifically binding PD-1. Administration of a therapeutically effective amount of the anti-PD-1 antibody of the present disclosure to a subject in need thereof can be used to treat diseases or disorders including inflammation, cancers and autoimmune diseases.

The antibodies of the present disclosure have multiple advantages, such as specific binding to PD-1, activation of PD-1 activity or PD-1 expression level, enhancement of immune suppression by PD-1 in a subject, inhibition of T lymphocytes, inhibition of the expression of cytokines such as IFN-γ by T lymphocytes, and prevention, treatment or adjuvant treatment of inflammation and autoimmune diseases.

Unless otherwise defined, all scientific and technical terms used herein have the same meaning as commonly understood by one of ordinary skill in the art to which the present disclosure belongs. In the event of a conflict, the definitions in the present specification shall prevail.

The terminology used herein is for the purpose of describing particular embodiments only and is not intended to limit the invention. Although only certain exemplary materials and methods are described herein, many methods and materials similar or equivalent to those described herein can be used in the practice of the present disclosure.

Unless the context clearly indicates otherwise, the singular forms "a", "an" and "the" used herein are intended to include the plural forms as well. In addition, the open-ended expressions "including" and "comprising" are interpreted as also including structural components or method steps that are not described, but it should be noted that the open-ended expressions also cover the situation consisting of only the described components and method steps (i.e., covering the situation of the closed expression "consisting of...").

In general, the term "about" is used herein to modify a numerical value above and below the stated value by a variance of 5%.

As used throughout, ranges are used as shorthand for describing each and every value that is within the range. Any value within the range, such as an integer value, a value that increases by one-tenth (when the end value of the range is one decimal place) or a value that increases by one-hundredth (when the end value of the range is two decimal places), can be selected as the terminus of the range. For example, range 1-10 is used to describe all numerical values within the range, such as 1, 2, 3, 4, 5, 6, 7, 8... 9, 9.1, 9.2, 9.3, 9.4, 9.5, 9.6, 9.7, 9.8, 9.9 and 10 (values that increase by one-tenth), and includes all subranges, such as 1-1.5, 2.0-3.0, 4.0-5.0, 6.0-7.0, 8.0-9.0, etc.

The term "antibody" as used herein may include whole antibodies and any antigen binding fragments (i.e., "antigen-binding portions") or single chains thereof. An "antibody" refers, in one embodiment, to a glycoprotein comprising at least two heavy (H) chains and two light (L) chains inter-connected by disulfide bonds, or an antigen binding fragment thereof. Each heavy chain comprises a heavy chain variable region (abbreviated herein as VH) and a heavy chain constant region. In certain naturally occurring IgG, IgD and IgA antibodies, the heavy chain constant region comprises three domains, CH1, CH2 and CH3. In certain naturally occurring antibodies, each light chain comprises a light chain variable region (abbreviated herein as VL) and a light chain constant region. The light chain constant region comprises one domain, CL. The VH and VL regions can be further subdivided into regions of hypervariability, termed complementarity determining regions (CDR), interspersed with regions that are more conserved, termed framework regions (FR). Each VH and VL comprises three CDRs and four framework regions (FRs), arranged from amino-terminus to carboxy-terminus in the following order: FR1, CDR1, FR2, CDR2, FR3, CDR3, FR4. The variable regions of the heavy and light chains contain a binding domain that interacts with an antigen. The constant regions of the antibodies may mediate the binding of the immunoglobulin to host tissues or factors, including various cells of the immune system (e.g., effector cells) and the first component (C1q) of the classical complement system. Unless otherwise indicated, an immunoglobulin may be from any of the commonly known isotypes, including but not limited to IgA, secretory IgA, IgG and IgM. The IgG isotype is divided into subclasses in certain species: IgG1, IgG2, IgG3 and IgG4 in humans, and IgG1, IgG2a, IgG2b and IgG3 in mice. Immunoglobulins, e.g., human IgG1, exist in several allotypes, which differ from each other in at most a few amino acids.

Unless otherwise indicated, "antibody" may include, for example, intact antibodies, single-chain antibodies (scFv), heavy chain antibodies, single domain antibodies, Fab fragments, Fab' fragments, F(ab')2, Fv, Fd fragments, bispecific antibodies (BsAb), etc.; monoclonal and polyclonal antibodies; chimeric and humanized antibodies; human and non-human antibodies; wholly synthetic antibodies, etc.

As used herein, an "antigen binding fragment" of an antibody refers to one or more fragments of an antibody that retain the ability to specifically bind to an antigen bound by an intact antibody. "Antigen-binding fragments" are any proteinaceous structure that may exhibit binding affinity for a particular antigen. Antigen-binding fragments include those provided by any known technique, such as enzymatic cleavage, peptide synthesis, and recombinant techniques. Some antigen-binding fragments are composed of portions of intact antibodies that retain antigen-binding specificity of the parent antibody molecule. For example, antigen-binding fragments may comprise at least one variable region (either a heavy chain or light chain variable region) or one or more CDRs of an antibody known to bind to a particular antigen. Examples of suitable antigen-binding fragments include, without limitation diabodies and single-chain molecules as well as Fab, F(ab')2, Fc, Fabc, and Fv molecules, single chain (Sc) antibodies, individual antibody light chains, individual antibody heavy chains, chimeric fusions between antibody chains or CDRs and other proteins, protein scaffolds, heavy chain monomers or dimers, light chain monomers or dimers, dimers consisting of one heavy and one light chain, a monovalent fragment consisting of the VL, VH, CL and CH1 domains, or a monovalent antibody as described in WO2007059782, bivalent fragments comprising two Fab fragments linked by a disulfide bridge at the hinge region, a Fd fragment consisting essentially of the VH and CH1 domains; a Fv fragment consisting essentially of the VL and VH domains of a single arm of an antibody, a dAb fragment which consists essentially of a VH domain and also called domain antibodies; camelid or nanobodies; an isolated complementarity determining region (CDR), and the like. All antibody isotypes may be used to produce antigen-binding fragments. Additionally, antigen-binding fragments may include non-antibody proteinaceous frameworks that may successfully incorporate polypeptide segments in an orientation that confers affinity for a given antigen of interest, such as protein scaffolds. Antigen-binding fragments may be recombinantly produced or produced by enzymatic or chemical cleavage of intact antibodies.

As used herein, the term sequence "identity" refers to a relationship between two or more polynucleotide sequences or two or more polypeptide sequences. When a position in a sequence is occupied by the same nucleic acid base or amino acid residue in the corresponding position of the comparison sequence, the sequence is called "identical" at that position. The percentage sequence "identity" is calculated by determining the number of positions where the same nucleic acid base or amino acid residue appears in the two sequences to produce the number of "identical" positions. Then, the number of "identical" positions is divided by the total number of positions in the comparison window and multiplied by 100 to produce the percentage of sequence "identity". The percentage of "identity" is determined by comparing the two optimally aligned sequences over the comparison window. In order to optimally align the sequences for comparison, the portion of the polynucleotide or polypeptide sequence in the comparison window may include additions or deletions called gaps, while the reference sequence remains constant. The optimal alignment is an alignment that produces the maximum possible number of "identical" positions between the reference sequence and the comparison sequence even with gaps. For example, the percent "identity" between two sequences can be determined using a version of the program "BLAST 2 Sequences" available from the National Center for Biotechnology Information (NCBI), which includes the programs BLASTN (for nucleotide sequence comparisons) and BLASTP (for polypeptide sequence comparisons), which are based on the algorithm of Karlin and Altschul (Proc. Natl. Acad. Sci. USA 90(12):5873-5877, 1993).

A "humanized" antibody refers to an antibody in which some, most or all of the amino acids outside the CDR domains of a non-human antibody are replaced with corresponding amino acids derived from human immunoglobulins. In one embodiment of a humanized form of an antibody, some, most or all of the amino acids outside the CDR domains have been replaced with amino acids from human immunoglobulins, whereas some, most or all amino acids within one or more CDR regions are unchanged. Small additions, deletions, insertions, substitutions or modifications of amino acids are permissible as long as they do not abrogate the ability of the antibody to bind to a particular antigen. A "humanized" antibody retains an antigenic specificity similar to that of the original antibody.

A "chimeric antibody" refers to an antibody in which the variable regions are derived from one species and the constant regions are derived from another species, such as an antibody in which the variable regions are derived from a mouse antibody and the constant regions are derived from a human antibody. A"hybrid" antibody refers to an antibody with heavy and light chains of different types, e.g., a mouse (parent) heavy chain and a humanized light chain, or vice versa.

The term "monoclonal antibody," as used herein, refers to an antibody that displays a single binding specificity and affinity for a particular epitope or a composition of antibodies in which all antibodies display a single binding specificity and affinity for a particular epitope.

As used herein, "isotype" refers to the antibody class (e.g., IgGl, IgG2, IgG3, IgG4, IgM, IgAl, IgA2, IgD, and IgE antibody) that is encoded by the heavy chain constant domain genes.

As used herein, the terms "antibody-dependent cell-mediated cytotoxicity" and "ADCC" refer to a cell-mediated process in which nonspecific cytotoxic cells expressing FcγRs (e.g., monocytes, such as natural killer (NK) cells and macrophages) recognize bound antibodies (or other proteins capable of binding to FcγRs) on target cells and subsequently cause target cell lysis. In principle, any effector cell with an activated FcγR can be triggered to mediate ADCC. The predominant cells that mediate ADCC are NK cells, which express only FcγRIII, while monocytes can express FcγRI, FcγRII and FcγRIII depending on their activation, localization or differentiation state.

**In one aspect,** the present disclosure provides a monoclonal antibody or antigen-binding fragment thereof that specifically binds PD-1, comprising heavy chain CDRs and light chain CDRs, wherein:
the amino acid sequence of heavy chain CDR1 comprises SEQ ID NOs: 15, 21, 27, 33, 39, 50 or 56, or a sequence having two or fewer substituted amino acids relative to SEQ ID NOs: 15, 21, 27, 33, 39, 50 or 56;
the amino acid sequence of heavy chain CDR2 comprises SEQ ID NOs: 16, 22, 28, 34, 40, 51 or 57, or a sequence having two or fewer substituted amino acids relative to SEQ ID NOs: 16, 22, 28, 34, 40, 51 or 57, and X in SEQ ID NO: 40 is any amino acid;
the amino acid sequence of heavy chain CDR3 comprises SEQ ID NOs: 17, 23, 29, 35, 44, 52 or 58, or a sequence having two or fewer substituted amino acids relative to SEQ ID NOs: 17, 23, 29, 35, 44, 52 or 58;
the amino acid sequence of light chain CDR1 comprises SEQ ID NOs: 18, 24, 30, 36, 45, 53 or 59, or a sequence having two or fewer substituted amino acids relative to SEQ ID NOs: 18, 24, 30, 36, 45, 53 or 59;
the amino acid sequence of light chain CDR2 comprises SEQ ID NOs: 19, 25, 31, 37, 46, 54 or 60, or a sequence having two or fewer substituted amino acids relative to SEQ ID NOs: 19, 25, 31, 37, 46, 54 or 60; and
the amino acid sequence of light chain CDR3 comprises SEQ ID NOs: 20, 26, 32, 38, 47, 55 or 61, or a sequence having two or fewer substituted amino acids relative to SEQ ID NOs: 20, 26, 32, 38, 47, 55 or 61, and X in SEQ ID NO: 47 is any amino acid.

In some embodiments, the substituted amino acids are conservative substitutions, for example, in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

**In one aspect,** the present disclosure provides an antibody that specifically binds PD-1, wherein the antibody comprises a heavy chain variable region and a light chain variable region,
the heavy chain variable region comprises heavy chain CDR1, heavy chain CDR2 and heavy chain CDR3,wherein the heavy chain CDR1, heavy chain CDR2 and heavy chain CDR3 respectively comprise amino acid sequences having at least 70%, 75%, 80%, 85%, 88%, 90%, 92%, 95%, 97%, 98%, 99% or 100% identity to one of the following groups:
   (1) SEQ ID NO: 15, SEQ ID NO: 16 and SEQ ID NO: 17;
   (2) SEQ ID NO: 21, SEQ ID NO: 22 and SEQ ID NO: 23;
   (3) SEQ ID NO: 27, SEQ ID NO: 28 and SEQ ID NO: 29;
   (4) SEQ ID NO: 33, SEQ ID NO: 34 and SEQ ID NO: 35;
   (5) SEQ ID NO: 39, SEQ ID NO: 40 and SEQ ID NO: 44;
   (6) SEQ ID NO: 50, SEQ ID NO: 51 and SEQ ID NO: 52; or
   (7) SEQ ID NO: 56, SEQ ID NO: 57 and SEQ ID NO: 58; and
the light chain variable region comprises light chain CDR1, light chain CDR2 and light chain CDR3, wherein the light chain CDR1, light chain CDR2 and light chain CDR3 respectively comprise amino acid sequences having at least 70%, 75%, 80%, 85%, 88%, 90%, 92%, 95%, 97%, 98%, 99% or 100% identity to one of the following groups:
   (1) SEQ ID NO: 18, SEQ ID NO: 19 and SEQ ID NO: 20;
   (2) SEQ ID NO: 24, SEQ ID NO: 25 and SEQ ID NO: 26;
   (3) SEQ ID NO: 30, SEQ ID NO: 31 and SEQ ID NO: 32;
   (4) SEQ ID NO: 36, SEQ ID NO: 37 and SEQ ID NO: 38;
   (5) SEQ ID NO: 45, SEQ ID NO: 46 and SEQ ID NO: 47;
   (6) SEQ ID NO: 53, SEQ ID NO: 54 and SEQ ID NO: 55; or
   (7) SEQ ID NO: 59, SEQ ID NO: 60 and SEQ ID NO: 61.

In some embodiments, the amino acid sequences of heavy chain CDR1, CDR2 and CDR3 as well as light chain CDR1, CDR2 and CDR3 are selected from one of the CDR combinations as shown in the following a to g:
a: the heavy chain CDR1, CDR2 and CDR3 sequences comprise SEQ ID NOs: 15-17 respectively; the light chain CDR1, CDR2 and CDR3 sequences comprise SEQ ID NOs: 18-20 respectively;
b: the heavy chain CDR1, CDR2 and CDR3 sequences comprise SEQ ID NOs: 21-23 respectively; the light chain CDR1, CDR2 and CDR3 sequences comprise SEQ ID NOs: 24-26 respectively;
c: the heavy chain CDR1, CDR2 and CDR3 sequences comprise SEQ ID NOs: 27-29 respectively; the light chain CDR1, CDR2 and CDR3 sequences comprise SEQ ID NOs: 30-32 respectively;
d: the heavy chain CDR1, CDR2 and CDR3 sequences comprise SEQ ID NOs: 33-35 respectively; the light chain CDR1, CDR2 and CDR3 sequences comprise SEQ ID NOs: 36-38 respectively;
e: the heavy chain CDR1, CDR2 and CDR3 sequences comprise SEQ ID NOs: 39, 40, 44 respectively; the light chain CDR1, CDR2 and CDR3 sequences comprise SEQ ID NOs: 45, 46, 47 respectively;
f: the heavy chain CDR1, CDR2 and CDR3 sequences comprise SEQ ID NOs: 50-52 respectively; the light chain CDR1, CDR2 and CDR3 sequences comprise SEQ ID NOs: 53-55 respectively; or
g: the heavy chain CDR1, CDR2 and CDR3 sequences comprise SEQ ID NOs: 56-58 respectively; the light chain CDR1, CDR2 and CDR3 sequences comprise SEQ ID NOs: 59-61 respectively.

In some embodiments, the heavy chain variable region comprises an amino acid sequence having at least 70%, 75%, 80%, 85%, 88%, 90%, 92%, 95%, 97%, 98%, 99% or 100% identity to any one of SEQ ID NOs: 1, 3, 5, 7, 9, 11 and 13.

In some embodiments, the light chain variable region comprises an amino acid sequence having at least 70%, 75%, 80%, 85%, 88%, 90%, 92%, 95%, 97%, 98%, 99% or 100% identity to any one of SEQ ID NOs: 2, 4, 6, 8, 10, 12 and 14.

In some embodiments, the heavy chain variable region and the light chain variable region are humanized.

In some embodiments, the humanized heavy chain variable region comprises an amino acid sequence having at least 70%, 75%, 80%, 85%, 88%, 90%, 92%, 95%, 97%, 98%, 99% or 100% identity to any one of SEQ ID NOs: 62, 64, 66, 68, 70, 71, 72 and 75.

In some embodiments, the humanized light chain variable region comprises an amino acid sequence having at least 70%, 75%, 80%, 85%, 88%, 90%, 92%, 95%, 97%, 98%, 99% or 100% identity to any one of SEQ ID NOs: 63, 65, 67, 69, 73, 74 and 76.

In some embodiments, the heavy chain comprises an amino acid sequence having at least 70%, 75%, 80%, 85%, 88%, 90%, 92%, 95%, 97%, 98%, 99% or 100% identity to SEQ ID NOs: 77, 78, 79, 82, 84, 86, 88 or 90.

In some embodiments, the light chain comprises an amino acid sequence having at least 70%, 75%, 80%, 85%, 88%, 90%, 92%, 95%, 97%, 98%, 99% or 100% identity to SEQ ID NOs: 80, 81, 83, 85, 87, 89 or 91.

In some embodiments, the antibody or antigen-binding fragment thereof is selected from an intact antibody, single-chain antibody (scFv), heavy chain antibody, single-domain antibody, Fab fragment, Fab' fragment, F(ab')2, Fv, Fd fragment, and bispecific antibody (BsAb).

In some embodiments, the antibody is a human antibody, humanized antibody or chimeric antibody.

In some embodiments, the antibody is isolated.

In some embodiments, the antibody is an isolated monoclonal antibody.

Also provided are "conservative sequence modifications" to the antibody sequence provided herein, i.e. nucleotide and amino acid sequence modifications that do not abrogate the binding of the antibody encoded by the nucleotide sequence or containing the amino acid sequence, to the antigen. For example, modifications can be introduced by standard techniques known in the art, such as site-directed mutagenesis and PCR-mediated mutagenesis. Conservative sequence modifications include conservative amino acid substitutions, in which the amino acid residue is replaced with an amino acid residue having a similar side chain. Families of amino acid residues having similar side chains have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine, tryptophan), nonpolar side chains (e.g., alanine, valine, leucine, isoleucine, proline, phenylalanine, methionine), beta-branched side chains (e.g., threonine, valine, isoleucine) and aromatic side chains (e.g., tyrosine, phenylalanine, tryptophan, histidine).

In some embodiments, the antibodies exhibit potent antibody-dependent cell-mediated cytotoxicity (ADCC).

**In one aspect,** the present disclosure provides a pharmaceutical composition comprising a therapeutically effective amount of the antibody and a pharmaceutically acceptable carrier, which may be an inert or physiologically active carrier. As used herein, the term "pharmaceutically acceptable carrier" includes any solvent, dispersion medium, coating, antibacterial agent, antifungal agent, etc. that is physiologically compatible. Examples of suitable carriers include water, saline, phosphate buffered saline, dextrose, glycerol, ethanol, etc., and any combination thereof.

**In one aspect,** the present disclosure provides an isolated nucleic acid molecule encoding the antibody.

**In one aspect,** the present disclosure provides an expression vector comprising the nucleic acid molecule.

**In one aspect,** the present disclosure provides a host cell, which expresses the antibody, and/or comprises the nucleic acid molecule or the expression vector.

**In one aspect,** the disclosure provides the antibody or the pharmaceutical composition for use in treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical composition is for administration in combination with one or more additional chemotherapeutic agents, radiotherapeutic agents, cytokines, or other antibodies.

**In one aspect,** the present disclosure provides use of the antibody or the pharmaceutical composition in the preparation of a medicament for treating a disease or disorder, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or the pharmaceutical composition is for administration in combination with one or more additional chemotherapeutic agents, radiotherapeutic agents, cytokines, or other antibodies.

**In one aspect,** the disclosure provides a method of treating a disease or disorder in a subject in need thereof, comprising administering the antibody or pharmaceutical composition in a therapeutically effective amount, wherein the disease or disorder is cancer or an autoimmune disease.

In some embodiments, the cancer is selected from the group consisting of colorectal cancer, colon cancer, renal cell carcinoma, breast cancer, epithelial squamous cell carcinoma, melanoma, myeloma, gastric cancer, brain cancer, lung cancer, pancreatic cancer, cervical cancer, ovarian cancer, liver cancer, bladder cancer, prostate cancer, testicular cancer, thyroid cancer, head and neck cancer, leukemia, and lymphoma.

In some embodiments, the antibody or pharmaceutical composition is administered in combination with one or more additional chemotherapeutic agents, radiotherapeutic agents, cytokines, or other antibodies.

As used herein, "administering" refers to the introduction of a composition comprising a therapeutic agent to a subject, using any of the various methods and delivery systems known to those skilled in the art. Routes of administration for the pharmaceutical composition of the present disclosure include intravenous, intramuscular, subcutaneous, intraperitoneal, spinal or other parenteral routes of administration, for example by injection or infusion. The phrase "parenteral administration" as used herein means modes of administration other than enteral and topical administration, usually by injection, and includes, without limitation, intravenous, intramuscular, intraarterial, intrathecal, intralymphatic, intralesional, intracapsular, intraorbital, intracardiac, intradermal, intraperitoneal, transtracheal, subcutaneous, subcuticular, intraarticular, subcapsular, subarachnoid, intraspinal, epidural and intrasternal injection and infusion, as well as in vivo electroporation. In some embodiments, the composition is administered by non-parenteral routes, in some embodiments, by oral administration. Other non-parenteral routes include topical, epidermal or mucosal route of administration, for example, intranasal, vaginal, rectal, sublingual or topical. Administering can also be performed, for example, once, a plurality of times, and/or over one or more extended periods.

As used herein, the term "effective amount" refers to the amount of an active agent required to provide a therapeutic and/or prophylactic benefit to a subject.

The "subject in need thereof" refers to any mammal, such as but not limited to humans, horses, cows, cats, mice, rabbits, rats, goats, etc. Preferably, the mammal is a human.

**In one aspect,** the present disclosure provides a kit comprising
a) the antibody or the pharmaceutical composition; and
b) instructions for use.

In some specific embodiments, the amino acid sequences of the heavy chain variable regions, light chain variable regions, heavy chains, light chains, heavy chain CDRs and light chain CDRs of the murine or humanized PD-1 agonist antibodies or functional fragments thereof provided by the present invention are shown in Table 1 below.

**Table 1 Sequence Information**

| Sequence Identifier | Description | Amino Acid Sequence |
|---|---|---|
| SEQ ID NO: 1 | 65A12 heavy chain variable region | EVKLEESGGGLVQPGGSMKLSYVASGFTFSNYWMNW VRQSPEKGLEWVAHIRLKSDNYATHYAESVRGRFTISR DDSKSSVYLQMNNLRAEDTGIYYCTGSHYGIAMDHW GQGTSVTVSS |
| SEQ ID NO: 2 | 65A12 light chain variable region | DVLMTQTPLSLPVSLGDQASISCRSSQNIVHINGVTYLE WYLQKPGQSPKLLIFKVSTRFSGVPDRFSGSGSGTDFTL KISRVEAEDLGVYYCFQGSYVPWTFGGGTKLEIK |
| SEQ ID NO: 3 | 69H4 heavy chain variable region | QVTLKESGPGILQPSQTLSLTCSFSGFSLSTFGMGVVWI RQPSGEGLEWLAHIWWDDDNYYNPALKSRLTISKDTS KNLVFLKIANVDTADTATYYCARIGDNYYFDYWGQG TTLTVSS |
| SEQ ID NO: 4 | 69H4 light chain variable region | DIVMSQSPSSQAVSVGEKVTMSCKSSQSLLHSSNQKNY LAWYQQKPGQSPKLLIYWASTRESGVPDRFTGSGSGT DFILTISSVKAEDLAVYYCQQYYNYPLTFGAGTKLELK |
| SEQ ID NO: 5 | 39A9 heavy chain variable region | EVQLQQSVAELVRPGASVKLSCTASGFNVKNTYFHWV NQRPEQGLEWIGRIDPANGNTKYAPKFQGKATITADTS SNTAYLQLSSLTSEDTAIYYCVFIYYGYDGDFDYWGQ GTTLTVSS |
| SEQ ID NO: 6 | 39A9 light chain variable region | DIQMNQSPSSLSASLGDTITITCHASQNINVWLSWYQQ KPGNIPKLLIYKASNLHTGVPSRFSGSGSGTGFSLTISSL QPEDIATYYCQQGQSYPLTFGSGTKLEIK |
| SEQ ID NO: 7 | 67B1 heavy chain variable region | EVQLQQSVAELVRPGASVKLSCTASAFNIKNTFMHWV KQRPEQGLEWIGRIGPANGNTKYDPKFLGKATITADTS SNTAYLQLSSLTSEDTAIYYCVLIYYDFDDNPMDYWG QGTSVTVSS |
| SEQ ID NO: 8 | 67B1 light chain variable region | DVLMTQTPLSLPVSLGDQASISCRSSQSVVHSNGNTYL EWYLQKPGQSPKLLIYRVSNRFSGVPDRFSGSGSGTDF TLKISRVEAEDLGVYYCFQGSHVPYTFGGGTKLEIK |
| SEQ ID NO: 9 | 54H8 heavy chain variable region | QVTLKESGPGILQPSQTLSLTCSFSGFSLSTFGMGVVWI RQPSGEGLEWLAHIWWDDDNYSNPALKSRLTISKDTS KNLVFLKIANVDTADNATYYCARIGDNYYFDYWGQG TTLTVSS |
| SEQ ID NO: 10 | 54H8 light chain variable region | DIVMSQSPSSQAVSVGEKVTLSCKSSQSLLHSSNQKNY LAWYQQKPGQSPKLLIYWASTRESGVPDRFTGSGSGT DFTLTISSVKAEDLAVYYCQQYYNYPLTFGAGTKLELK |
| SEQ ID NO: 11 | 48E7 heavy chain variable region | QVQLQQPGSELVRPGASVKLSCKASGYTFTNYWLHW VKQRHGQGLEWIGNIYPGRGTTNYDEKFKTKGTLTVD TSSSTAYMHLSSLTSEDSAVYYCIRSYYKYEDWYFDV WGAGTTVTVSS |
| SEQ ID NO: 12 | 48E7 light chain variable region | DIVLTQSPASLAVSLGQRATISCRASESVEYSGTNLMQ WYQQKPGQSPKLLIYAASNVESGVPARFSGSGSGTDFS LNIHPVDEDDIAMYFCQQSRKVPWTFGGGTKLEIR |
| SEQ ID NO: 13 | 4H6 heavy chain variable region | QVQLKESGPGLVAPSQSLSITCTVSGFSLTSYGVHWVR QPPGQGLEWLGVIWTGGNTDYNSALMSRLSINRDNSK SQVFLKMSSLQADDTAMYYCARASTGGYWGQGTLVT VSA |
| SEQ ID NO: 14 | 4H6 light chain variable region | DVLMTQTPLSLPVSLGDQASISCRSSQSIVHSDGNTYLE WYLQKPGQSPKLLISKVSNRFSGVPDRFSGSGSGTDFT LKISRVEAEDLGVYYCFQGSHLPLTFGAGTKLELK |
| SEQ ID NO: 15 | 65A12 heavy chain CDR1 | NYWMN |
| SEQ ID NO: 16 | 65A12 heavy chain CDR2 | HIRLKSDNYATHYAESVRG |
| SEQ ID NO: 17 | 65A12 heavy chain CDR3 | SHYGIAMDH |
| SEQ ID NO: 18 | 65A12 light chain CDR1 | RSSQNIVHINGVTYLE |
| SEQ ID NO: 19 | 65A12 light chain CDR2 | KVSTRFS |
| SEQ ID NO: 20 | 65A12 light chain CDR3 | FQGSYVPWT |
| SEQ ID NO: 21 | 69H4 heavy chain CDR1 | TFGMGVV |
| SEQ ID NO: 22 | 69H4 heavy chain CDR2 | HIWWDDDNYYNPALKS |
| SEQ ID NO: 23 | 69H4 heavy chain CDR3 | IGDNYYFDY |
| SEQ ID NO: 24 | 69H4 light chain CDR1 | KSSQSLLHSSNQKNYLA |
| SEQ ID NO: 25 | 69H4 light chain CDR2 | WASTRES |
| SEQ ID NO: 26 | 69H4 light chain CDR3 | QQYYNYPLT |
| SEQ ID NO: 27 | 39A9 heavy chain CDR1 | NTYFH |
| SEQ ID NO: 28 | 39A9 heavy chain CDR2 | RIDPANGNTKYAPKFQG |
| SEQ ID NO: 29 | 39A9 heavy chain CDR3 | IYYGYDGDFDY |
| SEQ ID NO: 30 | 39A9 light chain CDR1 | HASQNINVWLS |
| SEQ ID NO: 31 | 39A9 light chain CDR2 | KASNLHT |
| SEQ ID NO: 32 | 39A9 light chain CDR3 | QQGQSYPLT |
| SEQ ID NO: 33 | 67B1 heavy chain CDR1 | NTFMH |
| SEQ ID NO: 34 | 67B1 heavy chain CDR2 | RIGPANGNTKYDPKFLG |
| SEQ ID NO: 35 | 67B1 heavy chain CDR3 | IYYDFDDNPMDY |
| SEQ ID NO: 36 | 67B1 light chain CDR1 | RSSQSVVHSNGNTYLE |
| SEQ ID NO: 37 | 67B1 light chain CDR2 | RVSNRFS |
| SEQ ID NO: 38 | 67B1 light chain CDR3 | FQGSHVPYT |
| SEQ ID NO: 39 | 54H8 heavy chain CDR1 | TFGMGVV |
| SEQ ID NO: 40 | 54H8 heavy chain CDR2 | HIWWDDD**X**YSNPALKS |
| SEQ ID NO: 41 | 54H8 heavy chain CDR2 | HIWWDDDNYSNPALKS |
| SEQ ID NO: 42 | 54H8 heavy chain CDR2 | HIWWDDDQYSNPALKS |
| SEQ ID NO: 43 | 54H8 heavy chain CDR2 | HIWWDDDVYSNPALKS |
| SEQ ID NO: 44 | 54H8 heavy chain CDR3 | IGDNYYFDY |
| SEQ ID NO: 45 | 54H8 light chain CDR1 | KSSQSLLHSSNQKNYLA |
| SEQ ID NO: 46 | 54H8 light chain CDR2 | WASTRES |
| SEQ ID NO: 47 | 54H8 light chain CDR3 | QQYY**X**YPLT |
| SEQ ID NO: 48 | 54H8 light chain CDR3 | QQYYNYPLT |
| SEQ ID NO: 49 | 54H8 affinity - matured light chain CDR3 | QQYYTYPLT |
| SEQ ID NO: 50 | 48E7 heavy chain CDR1 | NYWLH |
| SEQ ID NO: 51 | 48E7 heavy chain CDR2 | NIYPGRGTTNYDEKFKT |
| SEQ ID NO: 52 | 48E7 heavy chain CDR3 | SYYKYEDWYFDV |
| SEQ ID NO: 53 | 48E7 light chain CDR1 | RASESVEYSGTNLMQ |
| SEQ ID NO: 54 | 48E7 light chain CDR2 | AASNVES |
| SEQ ID NO: 55 | 48E7 light chain CDR3 | QQSRKVPWT |
| SEQ ID NO: 56 | 4H6 heavy chain CDR1 | SYGVH |
| SEQ ID NO: 57 | 4H6 heavy chain CDR2 | VIWTGGNTDYNSALMS |
| SEQ ID NO: 58 | 4H6 heavy chain CDR3 | ASTGGY |
| SEQ ID NO: 59 | 4H6 light chain CDR1 | RSSQSIVHSDGNTYLE |
| SEQ ID NO: 60 | 4H6 light chain CDR2 | KVSNRFS |
| SEQ ID NO: 61 | 4H6 light chain CDR3 | FQGSHLPLT |
| SEQ ID NO: 62 | hu65A12 humanized heavy chain variable region | EVKLVESGGGLVKPGGSLRLSCAASGFTFSNYWMNW VRQAPGKGLEWVAHIRLKSDNYATHYAESVRGRFTIS RDDSKNTVYLQMNSLKTEDTAVYYCTGSHYGIAMDH WGQGTLVTVSS |
| SEQ ID NO: 63 | hu65A12 humanized light chain variable region | DVLMTQTPLSLSVTPGQPASISCRSSQNIVHINGVTYLE WYLQKPGQSPQLLIFKVSTRFSGVPDRFSGSGSGTDFTL KISRVEAEDVGVYYCFQGSYVPWTFGQGTKLEIK |
| SEQ ID NO: 64 | hu69H4 humanized heavy chain variable region | QVTLKESGPALVKPTQTLTLTCTFSGFSLSTFGMGVVW IRQPPGKALEWLAHIWWDDDNYYNPALKSRLTISKDT SKNLVVLTMTNMDPVDTATYYCARIGDNYYFDYWGQ GTLVTVSS |
| SEQ ID NO: 65 | hu69H4 humanized light chain variable region | DIVMTQSPDSLAVSLGERATINCKSSQSLLHSSNQKNY LAWYQQKPGQPPKLLIYWASTRESGVPDRFSGSGSGT DFILTISSLQAEDVAVYYCQQYYNYPLTFGGGTKVEIK |
| SEQ ID NO: 66 | hu39A9 humanized heavy chain variable region | EVQLVQSGAEVKKPGATVKISCKASGFNVKNTYFHWV QQAPGKGLEWIGRIDPANGNTKYAPKFQGRATITADTS TDTAYMELSSLRSEDTAVYYCVFIYYGYDGDFDYWGQ GTLVTVSS |
| SEQ ID NO: 67 | hu39A9 humanized light chain variable region | DIQMTQSPSSLSASVGDRVTITCHASQNINVWLSWYQQ KPGKAPKLLIYKASNLHTGVPSRFSGSGSGTGFSLTISSL QPEDFATYYCQQGQSYPLTFGGGTKVEIK |
| SEQ ID NO: 68 | hu67B1 humanized heavy chain variable region | EVQLVQSGAEVKKPGASVKVSCKASGFNIKNTFMHW VRQAPGQRLEWIGRIGPANGNTKYDPKFLGRATITADT SASTAYMELSSLRSEDTAVYYCVLIYYDFDDNPMDYW GQGTLVTVSS |
| SEQ ID NO: 69 | hu67B1 humanized light chain variable region | DVLMTQTPLSLSVTPGQPASISCRSSQSVVHSNGNTYLE WYLQKPGQSPQLLIYRVSNRFSGVPDRFSGSGSGTDFT LKISRVEAEDVGVYYCFQGSHVPYTFGQGTKLEIK |
| SEQ ID NO:70 | hu54H8 humanized heavy chain variable region | QVTLKESGPALVKPTQTLTLTCTFSGFSLSTFGMGVV WIRQPPGKALEWLAHIWWDDDNYSNPALKSRLTISK DTSKNLVFLTMTNMDPVDTATYYCARIGDNYYFDYW GQGTLVTVSS |
| SEQ ID NO:71 | hu54H8 humanized heavy chain variable region | QVTLKESGPALVKPTQTLTLTCTFSGFSLSTFGMGVV WIRQPPGKALEWLAHIWWDDDQYSNPALKSRLTISK DTSKNLVFLTMTNMDPVDTATYYCARIGDNYYFDYW GQGTLVTVSS |
| SEQ ID NO:72 | hu54H8 humanized heavy chain variable region | QVTLKESGPALVKPTQTLTLTCTFSGFSLSTFGMGVV WIRQPPGKALEWLAHIWWDDDVYSNPALKSRLTISK DTSKNLVFLTMTNMDPVDTATYYCARIGDNYYFDYW GQGTLVTVSS |
| SEQ ID NO:73 | hu54H8 humanized light chain variable region | DIVMTQSPDSLAVSLGERATINCKSSQSLLHSSNQKNY LAWYQQKPGQPPKLLIYWASTRESGVPDRFSGSGSGT DFTLTISSLQAEDVAVYYCQQYYNYPLTFGGGTKVEI K |
| SEQ ID NO:74 | hu54H8 humanized affinity - matured light chain variable region | DIVMTQSPDSLAVSLGERATINCKSSQSLLHSSNQKNY LAWYQQKPGQPPKLLIYWASTRESGVPDRFSGSGSGT DFTLTISSLQAEDVAVYYCQQYYTYPLTFGGGTKVEIK |
| SEQ ID NO:75 | hu4H6 humanized heavy chain variable region | QVQLQESGPGLVKPSETLSLTCTVSGFSLTSYGVHWVR QPPGKGLEWLGVIWTGGNTDYNSALMSRLTISRDNSK NQVSLKLSSVTAADTAVYYCARASTGGYWGQGTLVT VSS |
| SEQ ID NO:76 | hu4H6 humanized light chain variable region | DVVMTQTPLSLSVTPGQPASISCRSSQSIVHSDGNTYLE WYLQKPGQSPQLLISKVSNRFSGVPDRFSGSGSGTDFT LKISRVEAEDVGVYYCFQGSHLPLTFGQGTKLEIK |
| SEQ ID NO:77 | hu54H8 humanized heavy chain | QVTLKESGPALVKPTQTLTLTCTFSGFSLSTFGMGVV WIRQPPGKALEWLAHIWWDDDNYSNPALKSRLTISK DTSKNLVFLTMTNMDPVDTATYYCARIGDNYYFDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPDVFLFPPKPKDTLMISRTPEVT CVVVDVSHEDPEVKFNWYVDGVEVHNAKTKPREEQY NSTYRVVSVLTVLHQDWLNGKEYKCKVSNKALPAPEE KTISKAKGQPREPQVYTLPPSRDELTKNQVSLTCLVKG FYPSDIAVEWESNGQPENNYKTTPPVLDSDGSFFLYSK LTVDKSRWQQGNVFSCSVMHEALHNHYTQKSLSLSPG K |
| SEQ ID NO:78 | hu54H8 humanized heavy chain -N59Q | QVTLKESGPALVKPTQTLTLTCTFSGFSLSTFGMGVV WIRQPPGKALEWLAHIWWDDDQYSNPALKSRLTISK DTSKNLVFLTMTNMDPVDTATYYCARIGDNYYFDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPDVFLFPPKPKDTLMISRTPEVT CVVVDVSHEDPEVKFNWYVDGVEVHNAKTKPREEQY NSTYRVVSVLTVLHQDWLNGKEYKCKVSNKALPAPEE KTISKAKGQPREPQVYTLPPSRDELTKNQVSLTCLVKG FYPSDIAVEWESNGQPENNYKTTPPVLDSDGSFFLYSK LTVDKSRWQQGNVFSCSVMHEALHNHYTQKSLSLSPG K |
| SEQ ID NO:79 | hu54H8 humanized heavy chain -N59V | QVTLKESGPALVKPTQTLTLTCTFSGFSLSTFGMGVV WIRQPPGKALEWLAHIWWDDDVYSNPALKSRLTISK DTSKNLVFLTMTNMDPVDTATYYCARIGDNYYFDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPDVFLFPPKPKDTLMISRTPEVT CVVVDVSHEDPEVKFNWYVDGVEVHNAKTKPREEQY NSTYRVVSVLTVLHQDWLNGKEYKCKVSNKALPAPEE KTISKAKGQPREPQVYTLPPSRDELTKNQVSLTCLVKG FYPSDIAVEWESNGQPENNYKTTPPVLDSDGSFFLYSK LTVDKSRWQQGNVFSCSVMHEALHNHYTQKSLSLSPG K |
| SEQ ID NO:80 | hu54H8 humanized light chain | DIVMTQSPDSLAVSLGERATINCKSSQSLLHSSNQKNY LAWYQQKPGQPPKLLIYWASTRESGVPDRFSGSGSGT DFTLTISSLQAEDVAVYYCQQYYNYPLTFGGGTKVEI KRTVAAPSVFIFPPSDEQLKSGTASVVCLLNNFYPREAK VQWKVDNALQSGNSQESVTEQDSKDSTYSLSSTLTLS KADYEKHKVYACEVTHQGLSSPVTKSFNRGEC |
| SEQ ID NO: 81 | hu54H8 humanized affinity - matured light chain | DIVMTQSPDSLAVSLGERATINCKSSQSLLHSSNQKNY LAWYQQKPGQPPKLLIYWASTRESGVPDRFSGSGSGT DFTLTISSLQAEDVAVYYCQQYYTYPLTFGGGTKVEIK RTVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO: 82 | hu65A12 humanized heavy chain | EVKLVESGGGLVKPGGSLRLSCAASGFTFSNYWMNW VRQAPGKGLEWVAHIRLKSDNYATHYAESVRGRFTIS RDDSKNTVYLQMNSLKTEDTAVYYCTGSHYGIAMDH WGQGTLVTVSSASTKGPSVFPLAPSSKSTSGGTAALGC LVKDYFPEPVTVSWNSGALTSGVHTFPAVLQSSGLYSL SSVVTVPSSSLGTQTYICNVNHKPSNTKVDKKVEPKSC DKTHTCPPCPAPELLGGPDVFLFPPKPKDTLMISRTPEV TCVVVDVSHEDPEVKFNWYVDGVEVHNAKTKPREEQ YNSTYRVVSVLTVLHQDWLNGKEYKCKVSNKALPAP EEKTISKAKGQPREPQVYTLPPSRDELTKNQVSLTCLV KGFYPSDIAVEWESNGQPENNYKTTPPVLDSDGSFFLY SKLTVDKSRWQQGNVFSCSVMHEALHNHYTQKSLSLS PGK |
| SEQ ID NO: 83 | hu65A12 humanized light chain | DVLMTQTPLSLSVTPGQPASISCRSSQNIVHINGVTYLE WYLQKPGQSPQLLIFKVSTRFSGVPDRFSGSGSGTDFTL KISRVEAEDVGVYYCFQGSYVPWTFGQGTKLEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC |
| SEQ ID NO: 84 | hu69H4 humanized heavy chain | QVTLKESGPALVKPTQTLTLTCTFSGFSLSTFGMGVVW IRQPPGKALEWLAHIWWDDDNYYNPALKSRLTISKDT SKNLVVLTMTNMDPVDTATYYCARIGDNYYFDYWGQ GTLVTVSSASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKKVEPKSCDKT HTCPPCPAPELLGGPDVFLFPPKPKDTLMISRTPEVTCV VVDVSHEDPEVKFNWYVDGVEVHNAKTKPREEQYNS TYRVVSVLTVLHQDWLNGKEYKCKVSNKALPAPEEK TISKAKGQPREPQVYTLPPSRDELTKNQVSLTCLVKGF YPSDIAVEWESNGQPENNYKTTPPVLDSDGSFFLYSKL TVDKSRWQQGNVFSCSVMHEALHNHYTQKSLSLSPGK |
| SEQ ID NO: 85 | hu69H4 humanized light chain | DIVMTQSPDSLAVSLGERATINCKSSQSLLHSSNQKNY LAWYQQKPGQPPKLLIYWASTRESGVPDRFSGSGSGT DFILTISSLQAEDVAVYYCQQYYNYPLTFGGGTKVEIK RTVAAPSVFIFPPSDEQLKSGTASVVCLLNNFYPREAKV QWKVDNALQSGNSQESVTEQDSKDSTYSLSSTLTLSK ADYEKHKVYACEVTHQGLSSPVTKSFNRGEC |
| SEQ ID NO: 86 | hu39A9 humanized heavy chain | EVQLVQSGAEVKKPGATVKISCKASGFNVKNTYFHWV QQAPGKGLEWIGRIDPANGNTKYAPKFQGRATITADTS TDTAYMELSSLRSEDTAVYYCVFIYYGYDGDFDYWGQ GTLVTVSSASTKGPSVFPLAPSSKSTSGGTAALGCLVK DYFPEPVTVSWNSGALTSGVHTFPAVLQSSGLYSLSSV VTVPSSSLGTQTYICNVNHKPSNTKVDKKVEPKSCDKT HTCPPCPAPELLGGPDVFLFPPKPKDTLMISRTPEVTCV VVDVSHEDPEVKFNWYVDGVEVHNAKTKPREEQYNS TYRVVSVLTVLHQDWLNGKEYKCKVSNKALPAPEEK TISKAKGQPREPQVYTLPPSRDELTKNQVSLTCLVKGF YPSDIAVEWESNGQPENNYKTTPPVLDSDGSFFLYSKL TVDKSRWQQGNVFSCSVMHEALHNHYTQKSLSLSPGK |
| SEQ ID NO: 87 | hu39A9 humanized light chain | DIQMTQSPSSLSASVGDRVTITCHASQNINVWLSWYQQ KPGKAPKLLIYKASNLHTGVPSRFSGSGSGTGFSLTISSL QPEDFATYYCQQGQSYPLTFGGGTKVEIKRTVAAPSVF IFPPSDEQLKSGTASVVCLLNNFYPREAKVQWKVDNA LQSGNSQESVTEQDSKDSTYSLSSTLTLSKADYEKHKV YACEVTHQGLSSPVTKSFNRGEC |
| SEQ ID NO: 88 | hu67B1 humanized heavy chain | EVQLVQSGAEVKKPGASVKVSCKASGFNIKNTFMHW VRQAPGQRLEWIGRIGPANGNTKYDPKFLGRATITADT SASTAYMELSSLRSEDTAVYYCVLIYYDFDDNPMDYW GQGTLVTVSSASTKGPSVFPLAPSSKSTSGGTAALGCL VKDYFPEPVTVSWNSGALTSGVHTFPAVLQSSGLYSLS SVVTVPSSSLGTQTYICNVNHKPSNTKVDKKVEPKSCD KTHTCPPCPAPELLGGPDVFLFPPKPKDTLMISRTPEVT CVVVDVSHEDPEVKFNWYVDGVEVHNAKTKPREEQY NSTYRVVSVLTVLHQDWLNGKEYKCKVSNKALPAPEE KTISKAKGQPREPQVYTLPPSRDELTKNQVSLTCLVKG FYPSDIAVEWESNGQPENNYKTTPPVLDSDGSFFLYSK LTVDKSRWQQGNVFSCSVMHEALHNHYTQKSLSLSPG K |
| SEQ ID NO: 89 | hu67B1 humanized light chain | DVLMTQTPLSLSVTPGQPASISCRSSQSVVHSNGNTYLE WYLQKPGQSPQLLIYRVSNRFSGVPDRFSGSGSGTDFT LKISRVEAEDVGVYYCFQGSHVPYTFGQGTKLEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC |
| SEQ ID NO: 90 | hu4H6 humanized heavy chain | QVQLQESGPGLVKPSETLSLTCTVSGFSLTSYGVHWVR QPPGKGLEWLGVIWTGGNTDYNSALMSRLTISRDNSK NQVSLKLSSVTAADTAVYYCARASTGGYWGQGTLVT VSSASTKGPSVFPLAPSSKSTSGGTAALGCLVKDYFPEP VTVSWNSGALTSGVHTFPAVLQSSGLYSLSSVVTVPSS SLGTQTYICNVNHKPSNTKVDKKVEPKSCDKTHTCPPC PAPELLGGPDVFLFPPKPKDTLMISRTPEVTCVVVDVSH EDPEVKFNWYVDGVEVHNAKTKPREEQYNSTYRVVS VLTVLHQDWLNGKEYKCKVSNKALPAPEEKTISKAKG QPREPQVYTLPPSRDELTKNQVSLTCLVKGFYPSDIAVE WESNGQPENNYKTTPPVLDSDGSFFLYSKLTVDKSRW QQGNVFSCSVMHEALHNHYTQKSLSLSPGK |
| SEQ ID NO: 91 | hu4H6 humanized light chain | DVVMTQTPLSLSVTPGQPASISCRSSQSIVHSDGNTYLE WYLQKPGQSPQLLISKVSNRFSGVPDRFSGSGSGTDFT LKISRVEAEDVGVYYCFQGSHLPLTFGQGTKLEIKRTV AAPSVFIFPPSDEQLKSGTASVVCLLNNFYPREAKVQW KVDNALQSGNSQESVTEQDSKDSTYSLSSTLTLSKADY EKHKVYACEVTHQGLSSPVTKSFNRGEC |

The present inventors used a recombinant fragment of the PD-1 extracellular region as an antigen. After immunizing mice, mouse splenocytes were fused with myeloma cells to generate hybridoma cells. Through multiple rounds of cloning and screening of a large number of hybridomas, multiple monoclonal hybridoma cell lines were obtained. These hybridoma cell lines can secrete and generate monoclonal antibodies that specifically bind PD-1. Such monoclonal antibodies can potently activate PD-1 to suppress reporter signals of immune responses; cannot efficiently block the binding between PD-L1 and PD-1; and can effectively bind cynomolgus PD-1 protein.

Further, genes encoding antibody heavy and light chain variable regions were cloned by RT-PCR (Reverse Transcription-Polymerase Chain Reaction). Complementarity-determining region (CDR) grafting method was adopted to construct humanized antibodies in human IgG1 format.

In vitro functional assays demonstrated that humanized PD-1 agonist antibodies can specifically bind human and cynomolgus PD-1 proteins, and effectively activate PD-1 to suppress reporter signals of immune responses. The humanized PD-1 agonist antibodies can potently inhibit T cell cytokine secretion, T cell proliferation, and memory T cell proliferation.

In addition, such humanized antibodies exhibit significant antibody-dependent cellular cytotoxicity (ADCC). The present application further carried out affinity maturation and ADCC-enhancing modification on the hu54H8 antibody. The modified antibody (hu54H8-G1DE-M15) possesses enhanced specific binding to human PD-1 protein, elevated PD-1 activating activity, and stronger suppression of T cell cytokine release, T cell proliferation and memory T cell proliferation. Relative to the positive control antibodies Peresolimab from Eli Lilly (LY3462817) and Rosenilimab from Anaptysbio (ANB030), the modified hu54H8-G1DE-M15 disclosed herein exerts stronger inhibitory effects on T cell cytokine secretion, T cell proliferation and memory T cell proliferation. The sequence of the positive control antibody Peresolimab is disclosed in Patent Publication US20190270818A1 by Eli Lilly, and the sequence of the positive control antibody ANB030 is disclosed in Patent Publication WO2020247648A2 by Anaptysbio.

The present invention is further described with reference to the following nonlimiting Examples, which exemplify the development, characterization and efficacy of the above six antibodies 65A12, 69H4, 39A9, 67B1, 54H8 and 48E7.

### Example 1 Construction of Murine Hybridoma Antibodies

### Mouse immunization and hybridoma cell fusion

Human PD-1 extracellular region-mouse Fc fusion protein (PD-1-ECD-mFc) was used as the antigen. The antigen was fully emulsified with an equal volume of Complete Freund's Adjuvant (Sigma, Cat. No.: F5581), and subcutaneously administered to 6-8-week-old Balb/c mice (purchased from Joinn Laboratories (Suzhou) Co., Ltd.) at a dosage of 50 µg per mouse. Further immunizations were performed subcutaneously every 2 weeks for 3 times using the same dose of antigen, which was fully emulsified with Incomplete Freund's Adjuvant (Sigma, Cat. No.: F5506). Serum titers of the mice were measured after three rounds of immunization, and an intraperitoneal booster injection was administered 3 days prior to fusion. Mouse splenocytes and SP2/0 myeloma cells were mixed at a 4:1 ratio, using PEG Hybri-Max (Sigma, Cat. No.: 7181) as the fusion agent. The fused cells were added into the 96-well plate at 1×10⁵ cells per well, with 0.1 mL of 1×HAT Medium (Invitrogen, Cat. No.: 21060-017) per well. On Day 3, 0.1 mL of HT Medium (Invitrogen, Cat. No.: 11067-030) was supplemented to each well. On Day 7, the medium in the 96-well plate was aspirated and replaced with 0.2 mL of fresh HT Medium. Supernatants were collected on Day 9 for ELISA and FACS assay.

### ELISA screening for antibodies binding to PD-1-ECD

A 96-well plate (Corning, Cat. No.: 9018) was coated with human PD-1-ECD-hFc or cynomolgus PD-1-ECD-hFc fusion protein and incubated overnight at 4°C. The plate was washed 3 times with washing buffer (PBS+0.05% Tween 20), then blocked with PBS+1% BSA for 1 hour. After the 96-well plate was washed 3 times, hybridoma supernatants were added and incubated for 1 hour, followed by 3 washes. 100 µL of 1:10,000-diluted goat anti-mouse IgG secondary antibody (Thermo, Cat. No.: 31432) was added to each well and the plate was incubated at room temperature for 1 hour, followed by 3 washes. 100 µL TMB substrate (Biosubstrate Technologies, Cat. No.: ES-002) was added to each well for 3 min color development, and the reaction was terminated with 100 µL stop solution (2 N H₂SO₄) per well. OD₄₅₀ absorbance for each sample was determined using a microplate reader.

### Reporter assay for PD-1 activating response using hybridoma PD-1 agonist antibodies

The humanized PD-1 agonist antibody was serially diluted to prepare a 2-fold antibody dilution. 50 µL of the dilution was transferred to a 96-well plate, and 50 µL cell mixture was added, containing 1e5/well of Jurkat-PD-NFAT reporter cells, stimulatory agent OKT3 (ebioscience,cat.no:16-0037-85) at a final concentration of 10 ng/mL and 5e4/well of THP-1 cells. The plate was incubated at 37°C for 5 hours. Bright Glo reagent (Promega, Cat. No.: E2620) was added to each well for detection of reporter luminescence signals.

### ELISA screening for antibodies blocking PD-L1 binding to PD-1

Hybridoma antibodies were screened for blocking PD-L1 binding to PD-1 by ELISA assay. A 96-well ELISA plate was coated with PD-1-ECD-hFc and incubated overnight. The plate was washed 3 times with washing buffer (PBS+0.05% Tween 20), blocked with 200 µL blocking buffer for 1 hour, then washed 3 times. 100 µL hybridoma supernatant was added and incubated at room temperature for 1 hour. Subsequently, 100 µL PD-L1-Biotin was added to the sample well and incubated for 1 hour at room temperature. After washing 3 times, secondary antibody Avidin HRP(Invitrogen, Cat. No.: 18-4100-51) was added and incubated for 30 min. TMB was added for 3 min color development, and the reaction was terminated with 100 µL stop solution (2 N H₂SO₄) per well. OD₄₅₀ absorbance for each sample was determined using a microplate reader.

### Hybridoma subcloning

Hybridomas that can block PD-L1 binding to PD-1 were subcloned by the limiting dilution method, and then repeated ELISA and FACS screening were performed to obtain positive monoclonal hybridomas. The positive monoclonal hybridoma was cultured in 50 mL serum-free medium for 9 days, and then supernatant was collected by centrifugation. The monoclonal antibody was purified via Protein A affinity chromatography. Purified antibody samples were buffer-exchanged and concentrated using ultrafiltration centrifugal tubes (Millipore, Cat. No.: ACS500024). Protein concentration was determined via BCA assay, and the endotoxin level was measured using Tachypleus amebocyte lysate (TAL) (Xiamen Bioendo Technology Co., Ltd.).

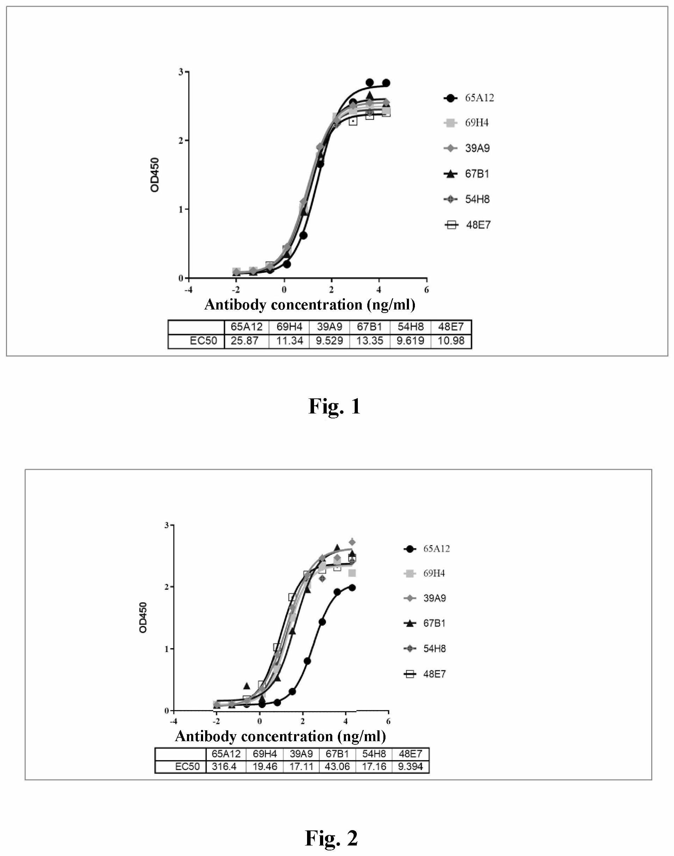

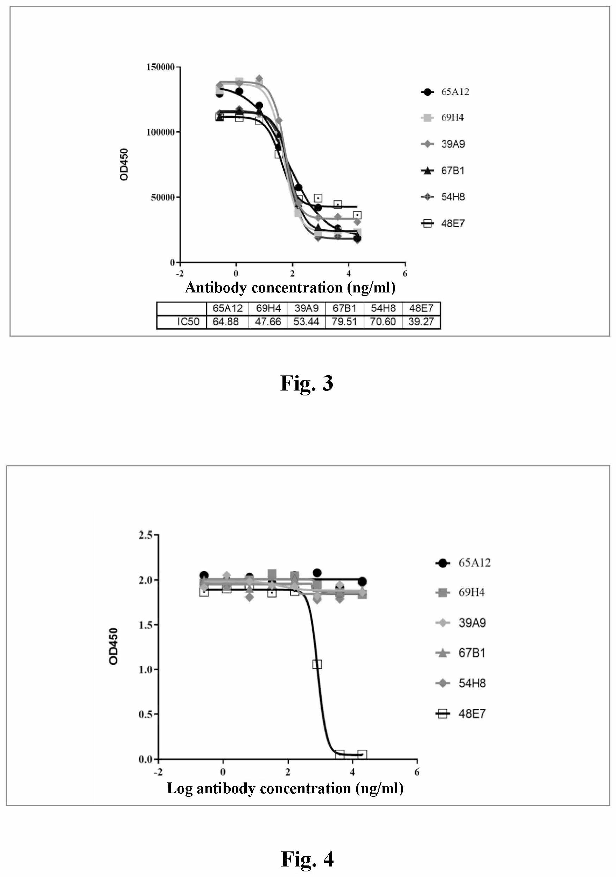

Binding of the purified hybridoma antibody sample to human or cynomolgus PD-1 was determined by ELISA and FACS. The activity of the antibody sample in activating PD-1 was determined using reporter assay. In addition, the activity of the antibody sample in blocking the binding between PD-L1 and PD-1 was determined by ELISA assay. The specific results are shown in Tables 2-4 and Figures 1-4. According to the information from the graphs and tables, the antibody samples exhibit favorable binding activity to PD-1 and good activating effect on PD-1. Furthermore, among the six purified antibodies, five show no blocking activity against the binding of PD-L1 to PD-1, whereas 48E7 possesses blocking activity against the binding of PD-L1 to PD-1.

**Table 2. Determination of PD-1 hybridoma antibody binding to human PD-1 by ELISA**

| Antibody | 65A12 | 69H4 | 39A9 | 67B1 | 54H8 | 48E7 |
|---|---|---|---|---|---|---|
| EC50 (ng/mL) | 25.87 | 11.34 | 9.529 | 13.35 | 9.619 | 10.98 |

**Table 3. Determination of PD-1 hybridoma antibody binding to cynomolgus PD-1 by ELISA**

| Antibody | 65A12 | 69H4 | 39A9 | 67B1 | 54H8 | 48E7 |
|---|---|---|---|---|---|---|
| EC50 (ng/mL) | 316.4 | 19.46 | 17.11 | 43.06 | 17.16 | 9.394 |

**Table 4. Determination of activating activity of hybridoma antibody to PD-1 by Reporter Assay**

| Antibody | 65A12 | 69H4 | 39A9 | 67B1 | 54H8 | 48E7 |
|---|---|---|---|---|---|---|
| IC50 (ng/mL) | 64.88 | 47.66 | 53.44 | 79.51 | 70.60 | 39.27 |

### Example 2

### Cloning of the PD-1 antibody variable region gene

The PD-1 monoclonal hybridoma cell line was lysed using TRIzon (Cwbiotech, Cat. No.: CW0580) to extract total RNA from hybridoma cells. The RNA from hybridoma cells was reverse transcribed into cDNA using a HiFi Script cDNA synthesis kit (Cwbiotech, Cat. No.: CW2569). Using cDNA as a template, the genes of the variable regions of the heavy and light chain of the antibody were amplified by PCR using degenerate primers (Kettleborough, et al., (1993) Eur. J. Immunology 23: 206-211; Strebe, et al., (2010) Antibody Engineering 1:3-14). The PCR amplification products were ligated into a T/A vector, transformed into DH5α competent cells, plated, and incubated overnight at 37 °C. Single clones were picked from the culture plate, expanded, and plasmids were extracted to determine the antibody gene sequence. Based on the antibody gene sequence, its complementarity determinants (CDRs) and framework regions were analyzed. The amino acid sequences of the variable regions and CDRs of the heavy and light chains of some antibodies are shown in Table 1.

### Example 3

### Humanization of murine PD-1 antibodies

Humanization of PD-1 antibodies was performed using complementarity-determining region grafting method. First, the IMGT database was searched for human germline antibody sequences with the highest homology to the light and heavy chain variable regions of murine antibodies 65A12, 69H4, 39A9, 67B1, 54H8, and 4H6. For the 65A12 antibody, the germline selected for humanization of light chain variable region was IGKV2-29*02, and for the heavy chain variable region, it was IGHV3-15*01. For the 69H4 antibody, the germline selected for humanization of light chain variable region was IGKV4-1*01, and for the heavy chain variable region, it was IGHV2-70*4. For the 39A9 antibody, the germline selected for humanization of light chain variable region was IGHV1-39*01, and for the heavy chain variable region, it was IGHV1-69-2*01. For the 67B1 antibody, the germline selected for humanization of light chain variable region was IGKV2-29*02, and for the heavy chain variable region, it was IGHV1-3*01. For the 54H8 antibody, the germline selected for humanization of light chain variable region was IGKV4-1*01, and for the heavy chain variable region, it was IGHV2-70*4. For the 4H6 antibody, the germline selected for humanization of light chain variable region was IGHV4-4*08, and for the heavy chain variable region, it was IGKV2-29*02. The CDR region of the murine antibody was retained, and the framework region sequence of the murine antibody was replaced with the framework region sequence of the human germline antibody. A structural model of the murine antibody was established, and the amino acids at each site in the framework region of the human antibody and the corresponding murine antibody were compared one by one. If using a human amino acid sequence at a certain site in the framework region did not cause damage or change to the spatial structure of the CDR region, then the human amino acid sequence was used at that site; otherwise, the corresponding murine sequence was used at that site (i.e., reverse-mutated to the murine sequence).

Based on structural simulations, Gln at position 3 of the humanized heavy chain of the 65A12 antibody was reverse-mutated to Lys, Gly at position 39 to Ala, and Leu at position 78 position to Val. Ile at position 2 of the humanized light chain of the 65A12 antibody was reverse-mutated to Val, Val at position 3 to Leu, and Tyr at position 49 to Phe. Gln at position 77 of the humanized heavy chain of the 69H4 antibody was reverse-mutated to Leu. Thr at position 72 of the humanized light chain of the 69H4 antibody was reverse-mutated to Ile. Val at position 24 of the humanized heavy chain of the 39A9 antibody was reverse-mutated to Ala, Tyr at position 27 to Phe, Thr at position 28 to Asn, Phe at position 29 to Val, Thr at position 30 to Lys, Met at position 48 to Ile, and Val at position 67 to Ala. Asp at position 70 of the humanized light chain of the 39A9 antibody was reverse-mutated to Gly and Thr at position 72 to Ser. Gln at position 1 of the humanized heavy chain of the 67B1 antibody was reverse-mutated to Glu; Tyr at position 27 to Phe; Thr at position 28 to Asn; Phe at position 29 to Ile; Thr at position 30 to Lys; Met at position 48 to Ile; Val at position 67 to Ala; and Arg at position 71 to Ala. Ile at position 2 of the humanized light chain of the 67B1 antibody was reverse-mutated to Val; and Val at position 3 to Leu. Gln at position 77 of the humanized heavy chain of the 54H8 antibody was reverse-mutated to Leu, Val at position 79 to Phe; and no reverse mutations were found in the humanized light chain of the 54H8 antibody. Gly at position 27 of the humanized heavy chain of the 4H6 antibody was reverse-mutated to Phe, Ile at position 29 to Leu, Ser at position 30 to Thr, Ile at position 37 to Val, Ile at position 48 to Leu, Val at position 67 to Leu, Val at position 71 to Arg, Thr at position 73 to Asn, and Phe at position 78 to Val. Ile at position 2 of the humanized light chain of the 4H6 antibody was reverse-mutated to Val and Tyr at position 49 to Ser.

The amino acid sequences of the variable regions of the heavy and light chain of the 65A12 humanized antibody are SEQ ID NO: 62 and SEQ ID NO: 63, respectively. The amino acid sequences of the variable regions of the heavy and light chain of the 69H4 humanized antibody are SEQ ID NO: 64 and SEQ ID NO: 65, respectively. The amino acid sequences of the variable regions of the heavy and light chain of the 39A9 humanized antibody are SEQ ID NO: 66 and SEQ ID NO: 67, respectively. The amino acid sequences of the variable regions of the heavy and light chain of the 67B1 humanized antibody are SEQ ID NO: 68 and SEQ ID NO: 69, respectively. The amino acid sequences of the variable regions of the heavy and light chain of the 54H8 humanized antibody are SEQ ID NO: 70/71/72 and SEQ ID NO: 73/74, respectively. The amino acid sequences of the variable regions of the heavy and light chain of the 4H6 humanized antibody are SEQ ID NO: 75 and SEQ ID NO: 76, respectively. Humanized antibodies 65A12, 69H4, 39A9, 67B1, 54H8, and 4H6 were constructed as the IgG1 subtype. Additionally, the humanized antibody 54H8 was constructed as an ADCC-enhanced version of the IgG1 subtype (containing S239D and I332E mutations in the heavy chain): SEQ ID NO: 77.

Nucleic acid fragments encoding the light and heavy chains of humanized antibodies 65A12, 69H4, 39A9, 67B1, 54H8, and 4H6 were synthesized and inserted into the expression vector pcDNA3.1. 200 mL of 293 cells (cell density 1 × 10⁶) were co-transfected with 0.1 mg each of the expression plasmids for antibody light and heavy chains. The cells were cultured at 37°C with shaking for 6 days. The supernatant was collected by centrifugation, and the humanized antibodies were purified using Protein A. The purified humanized antibodies were used for activity assays.

### Example 4

### Activity assay of humanized PD-1 antibody

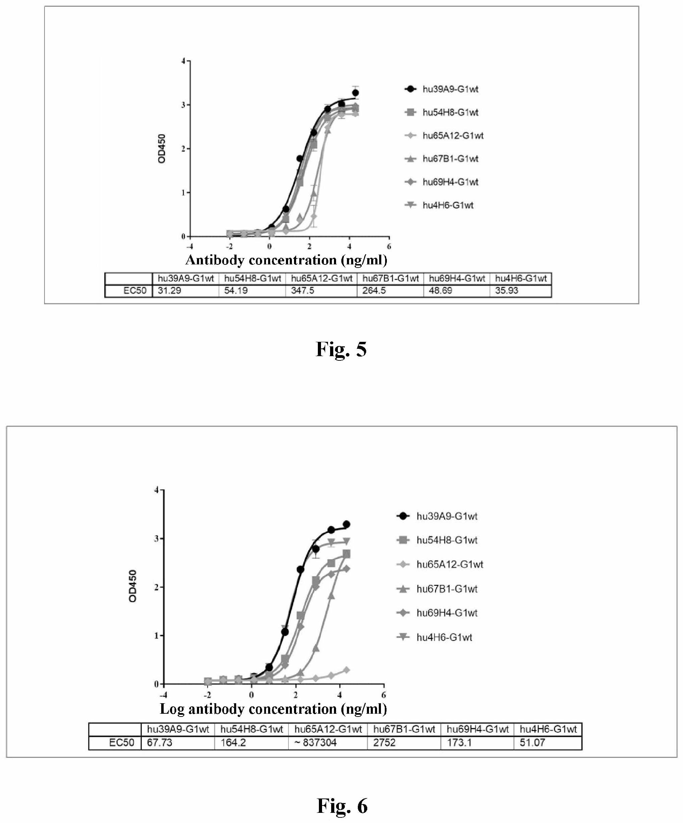

The binding of the purified humanized antibody samples to PD-1 was determined using ELISA and FACS, and the activating effect of humanized PD-1 antibodies on PD-1 was determined using reporter assay (See Example 1 for the specific method). The results of activity assay of the humanized antibody are shown in Table 5-7 and Figure 5-7. The results indicate that the PD-1 antigen-binding activity of the humanized PD-1 antibodies in this patent is generally good. As shown by the ELISA results, EC50 ranged from 31.29 ng/ml to 347.5 ng/ml. Among them, hu39A9, hu54H8, hu69H4, and hu4H6 showed relatively strong antigen-binding ability; their EC50 values were 31.29, 54.19, 48.69, and 35.93 ng/ml, respectively. The data from FACS analysis of PD-1 antigen binding ability showed that hu65A12 had weaker binding ability, while hu39A9 and hu4H6 had relatively better binding ability (EC50 values of 67.73 and 51.07 ng/ml, respectively); followed by hu54H8 and hu69H4, with EC50 values of 164.2 ng/ml and 173.1 ng/ml, respectively.

**Table 5. EC50 of PD-1 humanized antibody binding to PD-1 by ELISA.**

| Antibody | hu39A9 | hu54H8 | hu65A12 | hu67B1 | hu69H4 | hu4H6 |
|---|---|---|---|---|---|---|
| EC50 (ng/mL) | 31.29 | 54.19 | 347.5 | 264.5 | 48.69 | 35.93 |

**Table 6. PD-1 humanized antibody binding to cynomolgus PD-1 by ELISA.**

| Antibody | hu39A9 | hu54H8 | hu65A12 | hu67B1 | hu69H4 | hu4H6 |
|---|---|---|---|---|---|---|
| EC50 (ng/mL) | 67.73 | 164.2 | NA | 2752 | 173.1 | 51.07 |

**Table 7. Activating activity of humanized antibody against PD-1 by reporter assay.**

| Antibody | hu39A9 | hu54H8 | hu65A12 | hu67B1 | hu69H4 | hu4H6 |
|---|---|---|---|---|---|---|
| IC50 (ng/mL) | 46.73 | 64.04 | 278.7 | 87.55 | 57.30 | 45.47 |

### Example 5

### Determination of ADCC activity of humanized PD-1 agonist antibody

### Reporter assay for determining ADCC activity of humanized PD-1 antibody:

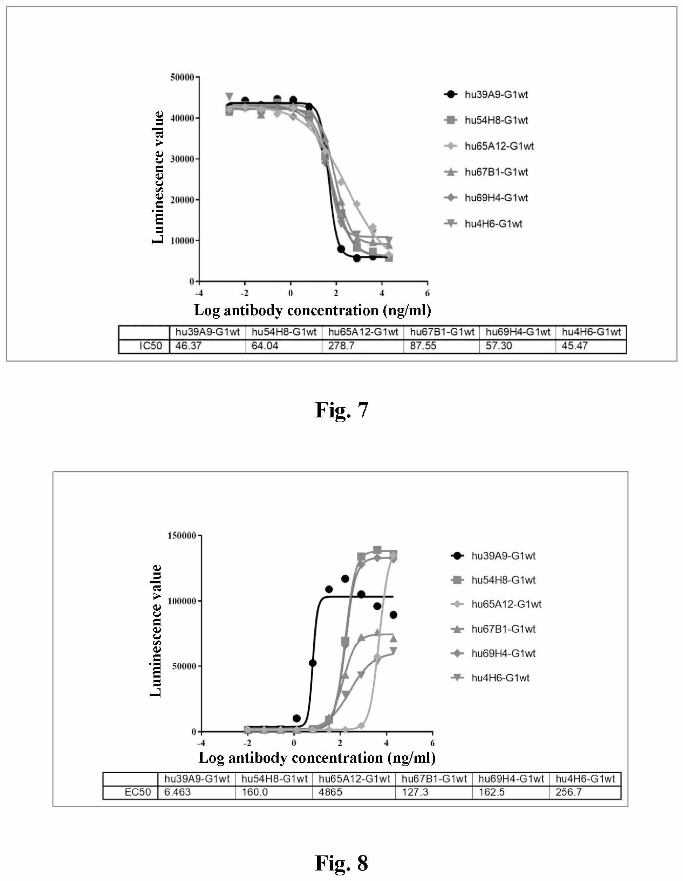

The humanized PD-1 agonist antibody was serially diluted to prepare a 2-fold antibody dilution. 50 µL of the dilution was transferred to a 96-well plate, and 50 µL cell mixture was added, containing 1e5/well of Jurkat-PD-cells and 1e5/well of Jurkat-CD16-NFAT cells. The plate was incubated at 37°C for 5 hours. Bright Glo reagent (Promega, Cat. No.: E2620) was added to each well for detection of reporter signal intensity. The assay results are shown in Table 8 and Figure 8. The results indicate that the humanized PD-1 antibody of this patent can induce ADCC production, with hu39A9 showing the strongest effect, followed by hu54H8, hu67B1, and hu69H4, while hu65A12 showed a relatively weaker effect.

**Table 8. ADCC activity of humanized PD-1 antibody by reporter assay**

| Antibody | hu39A9 | hu54H8 | hu65A12 | hu67B1 | hu69H4 | hu4H6 |
|---|---|---|---|---|---|---|
| EC50 (ng/mL) | 6.463 | 160.0 | 4865 | 127.3 | 162.5 | 256.7 |

### Example 6

The inhibition of humanized PD-1 agonist antibodies on T cell cytokine release, proliferation, and memory T cell proliferation.

### The effect of humanized PD-1 agonist antibody on cytokine secretion in mixed lymphocyte reactions:

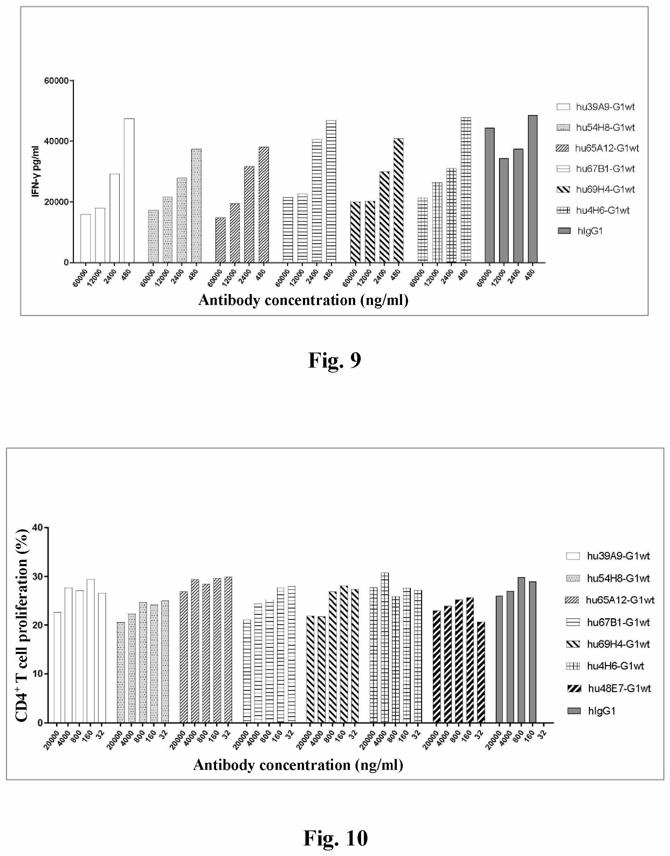

Thawed human PBMCs were seeded at a density of 2e5 cells /well in a 96-well plate, with a total volume of 200 µL. 40 ng/mL OKT3 (ebioscience, cat.no:16-0037-85) as the stimulatory agent was added, and the plate was incubated at 37°C for 2 days. The humanized PD-1 agonist antibody was serially diluted to prepare a 4- fold antibody dilution. 50 µL of this dilution was transferred to each well of the 96-well plate, and 150 µL of cell mixture was added, containing activated 2e5 cells/ well PBMCs, OKT3 at a final concentration of 25 ng/mL, and 5e4 cells/ well THP-1. The plate was incubated at 37°C for 2 days. The supernatant was collected, and the concentration of cytokines in the supernatant was determined using an IFN-γ ELISA kit. The assay results are shown in Figure 9. The results showed that the humanized PD-1 antibody in this patent generally exhibited a relatively significant inhibitory effect on the release of cytokines from T cells.

### The effect of humanized PD-1 agonist antibody on T cell proliferation:

The humanized PD-1 agonist antibody was serially diluted to prepare a 4-fold antibody dilution. 50 µL of this dilution was transferred to each well of a 96-well plate, and 150 µL of cell mixture was added, containing 1e5/ well CFSE- labeled human PBMC cells and SEB (Toxin Technology, cat.no:BT202) at a final concentration of 1ng/mL as the stimulatory agent. The plate was then incubated at 37°C for 3-4 days. Activated PBMC cells were collected, and 2.5 µL/well of CD4-PE-cy7 (Biolegend, cat.no:300512) was added. After incubation at 4 °C for 30 min, the cells were washed, and the percentage of T cell proliferation in CD4+ T cells was determined using flow cytometry. The assay results are shown in Figure 10. The results indicate that the humanized PD-1 antibody in this patent generally showed a significant inhibitory effect on T cell proliferation.

### The effect of humanized PD-1 agonist antibody on memory T cell proliferation:

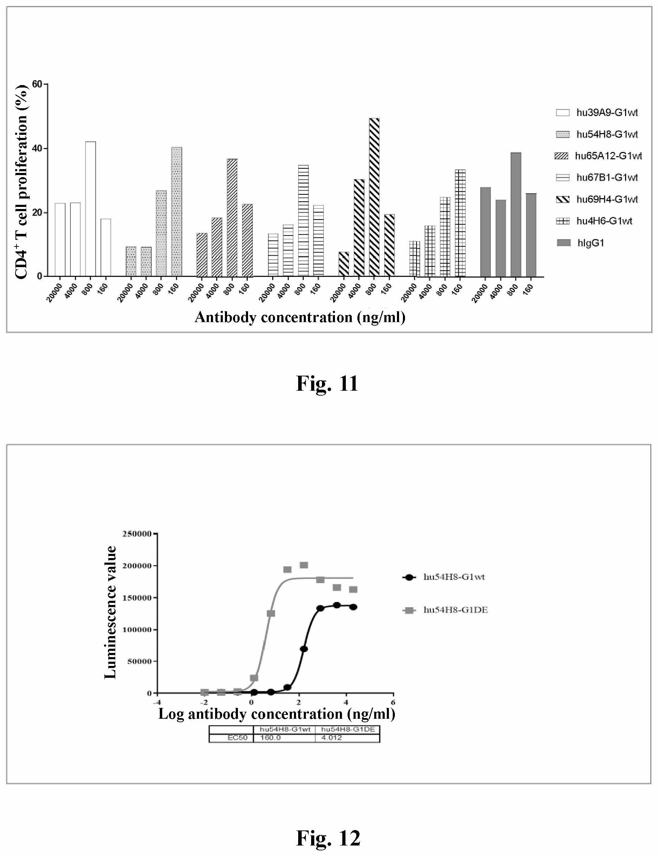

The humanized PD-1 agonist antibody was serially diluted to prepare a 4-fold antibody dilution. 50 µL of this dilution was transferred to each well of a 96-well plate, and 150 µL of cell mixture was added, containing 2e5/ well CFSE-labeled human PBMCs and CEFTA (Mabtech, cat.no: C34554) at a final concentration of 100 ng/mL as the stimulatory agent. The plate was then incubated at 37°C for 7-10 days. Activated PBMCs were collected, and 2.5 µL/ well of CD4-PE-cy7 (Biolegend, cat.no: 300512) was added. After incubation at 4 °C for 30 min, the cells were washed, and the percentage of memory T cell proliferation in CD4+ T cells was determined using flow cytometry. The assay results are shown in Figure 11. The results indicate that the humanized PD-1 antibody in this patent generally showed a significant inhibitory effect on memory T cell proliferation.

### Example 7

Modification of humanized PD-1 agonist antibody hu54H8 for ADCC activity enhancement and ADCC-enhanced version for further antibody construction.

Based on a comprehensive evaluation, especially considering primary cell assays, hu54H8 was selected as a candidate antibody for affinity maturation. Furthermore, we recognized that enhanced ADCC could promote PD- 1 activation and T- cell inhibition by humanized PD- 1 agonist antibodies. Therefore, hu54H8 antibody was modified for ADCC enhancement. We introduced two amino acid alterations (S239D and I332E mutations, SEQ ID NO: 77) into the heavy chain of the 54H8 IgG1 subtype. This alteration significantly enhanced the ADCC activity of hu54H8. Figure 12 shows that the ADCC-enhanced mutant hu54H8-G1DE (SEQ ID NO: 77 and SEQ ID NO: 80) exhibited stronger ADCC activity than the wild-type hu54H8-G1wt by ADCC reporter assay (see Example 5 for the specific method).

### Example 8

### Affinity maturation of humanized antibodies

(1) The humanized hu54H8 antibody was converted into the corresponding single-chain antibody (scFv) form. The encoded nucleic acid sequence was optimized using codons preferred by *E. coli*, and then synthesized by a gene synthesis company and cloned into the phage display vector PAK100. Using the GeneMorph II Random Mutagenesis Kit (Cat NO:200550) from Agilent, the encoded nucleic acid sequence was randomly mutated by PCR according to the instructions. The PCR product and the phage display vector PAK100 were digested with restriction endonucleases NcoI and EcoRI. The nucleic acid fragments were purified and recovered. The PCR product fragments were ligated to the phage display vector using DNA ligase. The ligation product was then electroporated into competent XL-Blue *E. coli* to construct a random mutant library of the hu54H8 antibody. 10 µl of transformation solution was serially diluted and plated, whereas the remaining transformation solution was spread onto a 15 cm petri dish, which was incubated overnight at 37 °C. The following day, colonies were scraped from the petri dish and frozen. A portion of the frozen bacteria was inoculated into 200 ml of 2YT medium and incubated until the OD600 reached 0.5. 10¹² M13KO7 was added for superinfection and the culture was incubated overnight at 32°C. The next day, the phage library was concentrated to 1 ml by PEG/NaCl precipitation.
(2) A 500 µl phage library was mixed with biotin-labeled PD-1 protein and incubated at room temperature for 2 hours. Then, 50 µl of streptavidin magnetic beads blocked with bovine serum albumin were added, and the mixture was incubated at room temperature for 30 minutes. The mixture was placed on a magnetic rack, the liquid was aspirated, and the mixture was repeatedly washed with 1 ml of PBST to remove weakly binding phages. Unlabeled PD-1 protein was then added and the mixture was incubated overnight for competition. The next day, the mixture was washed repeatedly with 1 ml of PBST. Finally, the phages adsorbed on the magnetic beads were digested with trypsin and used to infect logarithmically growing *E. coli* XL-Blue. After superinfection, a new batch of phages was produced and concentrated for the next round of panning. The above steps were repeated 5 times to enrich high-affinity clones.
(3) scFv phage screening by competitive ELISA. The last round of panned monoclonal *E. coli* were inoculated into a 96 -well deep-well plate, with 300 µl of 2YT medium containing ampicillin and M13-KO7 helper phage added to each well. The plate was incubated overnight at 37°C to prepare monoclonal scFv phages. The culture supernatant containing scFv phages was diluted 5- fold with PBST buffer, and 10 nM PD-1 antigen or BSA was added. The plate was incubated at room temperature for 1 hour. The mixture was then transferred to a 96 -well ELISA plate coated with PD-1 and blocked with BSA. The plate was incubated at room temperature for 15 minutes and then washed 8 times with PBST. Anti-M13 antibody conjugated with horseradish peroxidase was added, and incubation was continued for 30 min. The plate was washed with PBST, TMB was added for color development, and the color development was stopped with 1.0 M H₂SO₄. The absorbance of the samples at 450 nm was measured. Monoclonal *E. coli* exhibiting low absorbance were selected for sequencing. Compared with the humanized antibody sequence, the mutations in the heavy and light chain sequences of the antibody finally selected after affinity maturation are shown in Table 9.

**Table 9. Mutations in the heavy and light chain sequences of humanized PD-1 antibody before and after affinity maturation.**

| Heavy and light chain of humanized antibody | Position | humanized | Affinity-matured |
|---|---|---|---|
| hu54H8-VL | LCDR3 | SEQ ID NO: 48 | SEQ ID NO: 49 |
| hu54H8-VH-DE | | Unchanged | |

### Example 9

### Determination of the binding ability of affinity-matured humanized PD-1 agonist antibody to PD-1

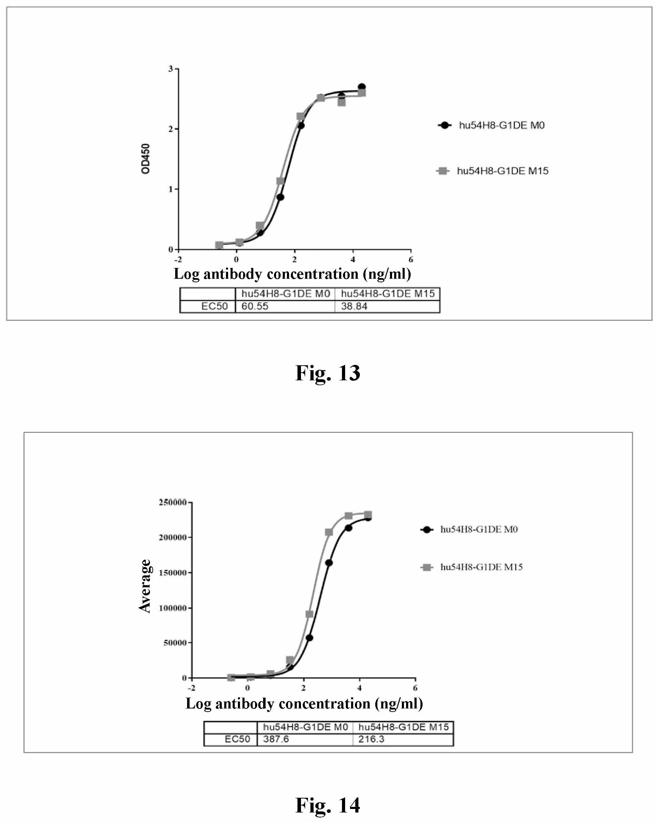

The binding of purified humanized antibody samples to PD-1 was determined using ELISA and FACS (See Example 1 for the specific method). The results are shown in Table 10 and Figures 13 and 14. The results indicate that, compared with the humanized hu54H8 antibody, by mutating Asn at position 99 of its light chain to Thr (light chain SEQ ID NO: 81), the antigen-binding ability of the antibody was increased by 1.6 times (ELISA assay) or 1.79 times (FACS assay).

**Table 10. Binding activity of humanized and affinity-matured PD-1 antibodies to PD-1-mFc and their PD-1 activating activity.**

| | Sequence Identifier | ELISA EC50 | FACS EC50 |
|---|---|---|---|
| hu54H8-G1DE-M0 | SEQ ID NO: 77/80 | 60.55 | 387.6 |
| hu54H8-G1DE-M15 | SEQ ID NO: 77/81 | 38.84 | 216.3 |

### Example 10

### Determination of the PD-1 activating activity by affinity-matured humanized PD-1 agonist antibody

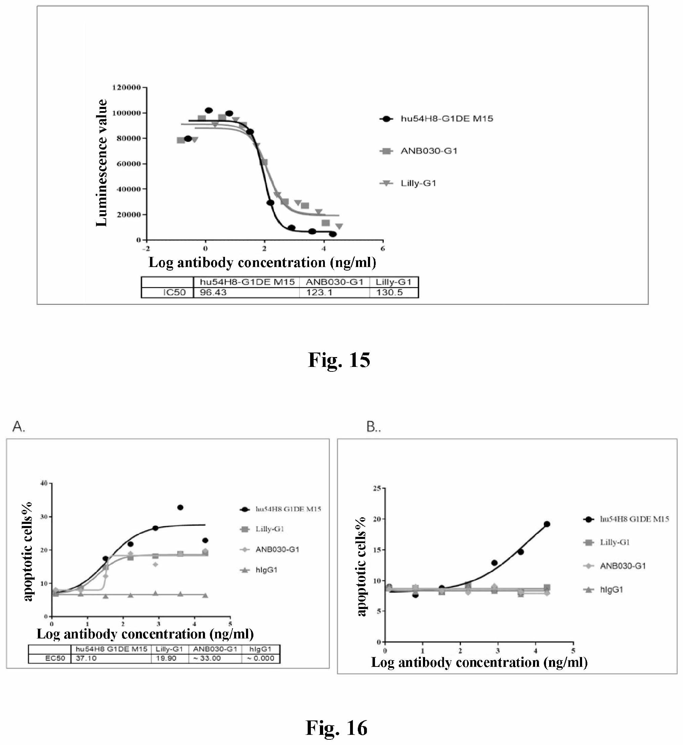

The activating effect of humanized PD-1 antibody on PD-1 was determined using reporter assay (See Example 1 for the specific method). The results are shown in Figure 15. The results indicate that the affinity-matured humanized PD-1 agonist antibody, hu54H8-G1DE-M15, compared with the positive control antibody Lilly-G1 (Peresolimab, LY3462817; Eli Lilly) and the positive control antibody ANB030 (Anaptysbio), had a stronger effect in promoting PD-1 activity and thus exerted a more significant inhibitory effect on T-cell activation.

### Example 11

### Determination of T-cell killing activity of affinity-matured humanized PD-1 antibody

Killer cells were assayed using the FACS method. Donor A T cells were induced to express PD1 with 10 ng/ml SEB (Toxin technology, Cat. No: BT202). After 3-5 days, cells were collected as target cells, or uninduced Jurkat-PD-1 cells were used as target cells. The cells were then labeled with CFSE and ready for use. The PD-1 agonist antibody was then serially diluted to prepare a 4- fold antibody dilution. 50 µL of this dilution was transferred to a 96-well plate, and 150 µL of cell mixture was added. The ratio of Donor B PBMCs as the effector cells to CFSE-labeled Donor A T cells or Jurkat-PD1 cells was 25:1. The cells were incubated at 37°C for 4 hours (Jurkat-PD-1 cells as target cells) or 16 hours (induced T cells as target cells). After incubation with apoptosis-detection reagent 7-AAD (Biolegend, Cat. No: 420404) for 15 minutes, the proportion of apoptotic cells in CFSE- positive cells was determined by flow cytometry. The results are shown in Figures 16A (Jurkat-PD-1 as the target cell) and 16B (induced T cells as the target cell). The results indicate that the affinity-matured humanized PD-1 agonist antibody, hu54H8-G1DE-M15, exhibited stronger ADCC activity than the positive control antibodies Lilly-G1 (Peresolimab, LY3462817; Eli Lilly) and ANB030 (Anaptysbio), especially when induced T cells were used as the target cells. hu54H8-G1DE-M15 showed significant ADCC activity, while Lilly-G1 and ANB030 did not exhibit significant ADCC activity.

### Example 12

The inhibitory effect of the affinity-matured humanized PD-1 agonist antibodies on T cell cytokine release, proliferation, and memory T cell proliferation.

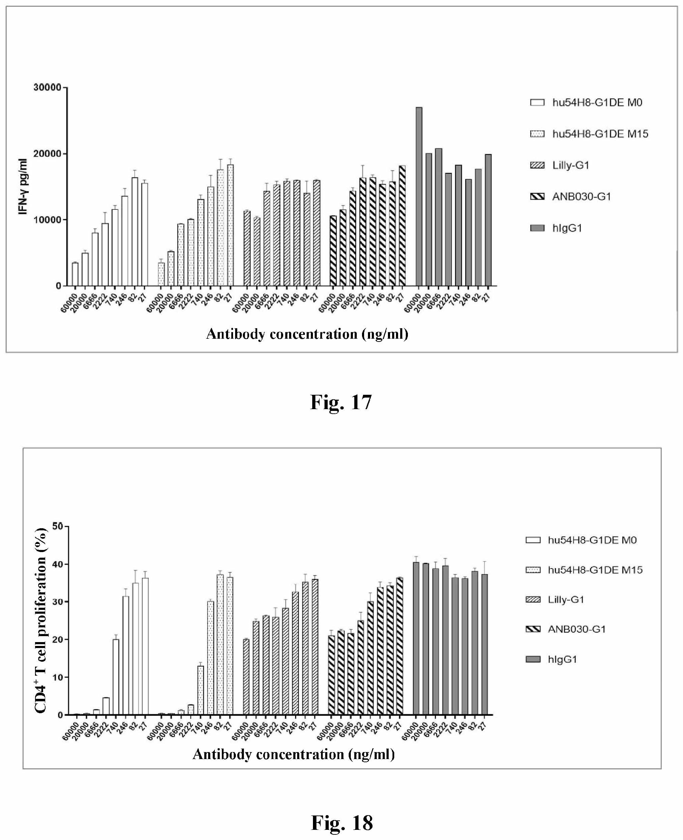

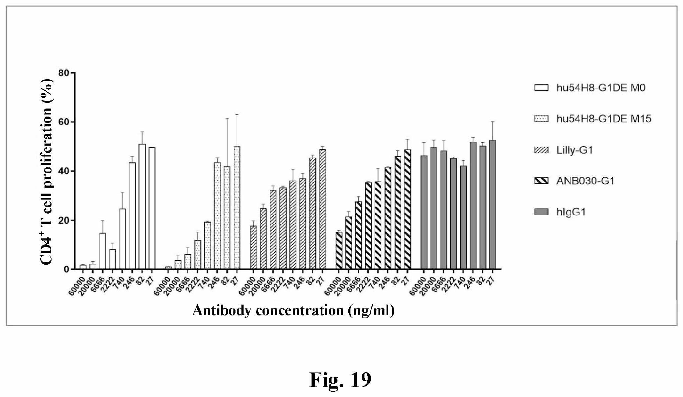

The effect of affinity-matured humanized PD-1 agonist antibody, hu54H8-G1DE-M15, on cytokine secretion in mixed lymphocyte reactions; the effect of affinity-matured humanized PD-1 agonist antibody on T cell proliferation; and the inhibition of memory T cell proliferation by humanized PD-1 agonist antibody were determined (See Example 6 for specific methods). In general, the affinity-matured humanized PD-1 agonist antibody showed improvement in all three of the above functions (see Figures 17, 18, and 19).

Furthermore, compared with the positive control antibody Lilly-G1 (Peresolimab, LY3462817; Eli Lilly) and the positive control antibody ANB030 (Anaptysbio), the humanized PD-1 agonist antibody of the present invention exhibited a stronger and more significant inhibitory effect on T cell cytokine release, proliferation, and memory T cell proliferation (see Figures 15, 16, and 17).

### Example 13

The amino acid at position 59 in the CDR2 sequence of the heavy chain of hu54H8-G1DE-M15 is asparagine (N59, heavy chain sequence is SEQ ID NO: 77). According to our in-silico analysis, there is a possibility of glycosylation. To reduce the impact of glycosylation, we therefore introduced two point mutations at this site, N59Q and N59V (The corresponding heavy chain sequences are SEQ ID NO: 78 and SEQ ID NO: 79, respectively). After antigen binding ability and functional analysis (see Example 1 for specific methods), these two mutants did not show significant changes in binding ability and functional activity (Table 11). Therefore, the three sequences hu54H8-G1DE-M15, hu54H8-G1DE-M15-N59Q and hu54H8-G1DE-M15-N59V can achieve the same technical effect.

**Table 11. Results of antigen-binding ability and functional activity analysis of humanized hu54H8-G1DE-M15-N59Q and hu54H8-G1DE-M15-N59V mutants.**

| Relevant analysis | hu54H8-G1DE-M15 | hu54H8-G1DE-M15-N59Q | hu54H8-G1DE-M15-N59V |
|---|---|---|---|
| Antigen binding analysis ( ELISA ) - EC 50(ng/ml) | 216.3 | 230.5 | 250.1 |
| PD1 reporter assay with PBMC-IC50 (ng/ml) | 6.037 | 5.602 | 5.196 |
| PD1 reporter assay with THP1-IC50 (ng/ml) | 28.51 | 33.79 | 27.96 |

The results of the above Examples indicate that the monoclonal antibody or its antigen-binding fragment described in this invention, or monoclonal antibody conjugates comprising the monoclonal antibody or its antigen-binding fragment described in this invention, have broad prospects in medicaments that bind to PD-1, medicaments that activate PD-1 activity or PD-1 expression levels, medicaments that enhance immune suppression by PD-1 in a subject, medicaments that inhibit T lymphocytes, medicaments that inhibit the expression of cytokines such as IFN-γ by T lymphocytes, and medicaments for the prevention, treatment, or adjuvant treatment of inflammation and autoimmune diseases.

The above are only preferred embodiments of the present invention. It should be noted that for those skilled in the art, several improvements can be made without departing from the principle of the present invention, and these improvements should also be considered within the scope of protection of the present invention.

## Claims

1. A monoclonal antibody or antigen-binding fragment thereof that specifically binds PD-1, comprising heavy chain CDRs and light chain CDRs, wherein:
the amino acid sequence of heavy chain CDR1 comprises SEQ ID NOs: 15, 21, 27, 33, 39, 50 or 56, or a sequence having two or fewer substituted amino acids relative to SEQ ID NOs: 15, 21, 27, 33, 39, 50 or 56;
the amino acid sequence of heavy chain CDR2 comprises SEQ ID NOs: 16, 22, 28, 34, 40, 51 or 57, or a sequence having two or fewer substituted amino acids relative to SEQ ID NOs: 16, 22, 28, 34, 40, 51 or 57, wherein X in SEQ ID NO: 40 is any amino acid;
the amino acid sequence of heavy chain CDR3 comprises SEQ ID NOs: 17, 23, 29, 35, 44, 52 or 58, or a sequence having two or fewer substituted amino acids relative to SEQ ID NOs: 17, 23, 29, 35, 44, 52 or 58;
the amino acid sequence of light chain CDR1 comprises SEQ ID NOs: 18, 24, 30, 36, 45, 53 or 59, or a sequence having two or fewer substituted amino acids relative to SEQ ID NOs: 18, 24, 30, 36, 45, 53 or 59;
the amino acid sequence of light chain CDR2 comprises SEQ ID NOs: 19, 25, 31, 37, 46, 54 or 60, or a sequence having two or fewer substituted amino acids relative to SEQ ID NOs: 19, 25, 31, 37, 46, 54 or 60; and
the amino acid sequence of light chain CDR3 comprises SEQ ID NOs: 20, 26, 32, 38, 47, 55 or 61, or a sequence having two or fewer substituted amino acids relative to SEQ ID NOs: 20, 26, 32, 38, 47, 55 or 61, wherein X in SEQ ID NO: 47 is any amino acid.

2. The antibody or antigen-binding fragment thereof according to claim 1, wherein X in the amino acid sequence of SEQ ID NO: 40 is N, Q or V.

3. The antibody or antigen-binding fragment thereof according to claim 1, wherein X in the amino acid sequence of SEQ ID NO: 47 is N or T.

4. The antibody or antigen-binding fragment thereof according to claim 1, wherein the amino acid sequences of heavy chain CDR1, heavy chain CDR2, heavy chain CDR3, light chain CDR1, light chain CDR2 and light chain CDR3 are selected from one of the CDR combinations as shown in the following a to g:
a: the heavy chain CDR1, CDR2 and CDR3 sequences comprise SEQ ID NOs: 15-17 respectively; the light chain CDR1, CDR2 and CDR3 sequences comprise SEQ ID NOs: 18-20 respectively;
b: the heavy chain CDR1, CDR2 and CDR3 sequences comprise SEQ ID NOs: 21-23 respectively; the light chain CDR1, CDR2 and CDR3 sequences comprise SEQ ID NOs: 24-26 respectively;
c: the heavy chain CDR1, CDR2 and CDR3 sequences comprise SEQ ID NOs: 27-29 respectively; the light chain CDR1, CDR2 and CDR3 sequences comprise SEQ ID NOs: 30-32 respectively;
d: the heavy chain CDR1, CDR2 and CDR3 sequences comprise SEQ ID NOs: 33-35 respectively; the light chain CDR1, CDR2 and CDR3 sequences comprise SEQ ID NOs: 36-38 respectively;
e: the heavy chain CDR1, CDR2 and CDR3 sequences comprise SEQ ID NOs: 39, 40, 44 respectively; the light chain CDR1, CDR2 and CDR3 sequences comprise SEQ ID NOs: 45, 46, 47 respectively;
f: the heavy chain CDR1, CDR2 and CDR3 sequences comprise SEQ ID NOs: 50-52 respectively; the light chain CDR1, CDR2 and CDR3 sequences comprise SEQ ID NOs: 53-55 respectively; or
g: the heavy chain CDR1, CDR2 and CDR3 sequences comprise SEQ ID NOs: 56-58 respectively; the light chain CDR1, CDR2 and CDR3 sequences comprise SEQ ID NOs: 59-61 respectively.

5. The antibody or antigen-binding fragment thereof according to claim 1, comprising a heavy chain variable region and a light chain variable region, wherein the amino acid sequence of the heavy chain variable region is selected from any one of SEQ ID NOs: 1, 3, 5, 7, 9, 11 and 13; and the amino acid sequence of the light chain variable region is selected from any one of SEQ ID NOs: 2, 4, 6, 8, 10, 12 and 14.

6. The antibody or antigen-binding fragment thereof according to claim 5, wherein the heavy chain variable region and the light chain variable region are humanized.

7. The antibody or antigen-binding fragment thereof according to claim 6, wherein the amino acid sequence of the humanized heavy chain variable region is selected from any one of SEQ ID NOs: 62, 64, 66, 68, 70, 71, 72 and 75; and the amino acid sequence of the humanized light chain variable region is selected from any one of SEQ ID NOs: 63, 65, 67, 69, 73, 74 and 76.

8. The antibody or antigen-binding fragment thereof according to claim 7, comprising a heavy chain and a light chain, wherein:
the amino acid sequence of the heavy chain is SEQ ID NO: 77, and the amino acid sequence of the light chain is selected from SEQ ID NO: 80 or SEQ ID NO: 81; or
the amino acid sequence of the heavy chain is SEQ ID NO: 78 or SEQ ID NO: 79, and the amino acid sequence of the light chain is SEQ ID NO: 81; or
the amino acid sequence of the heavy chain is SEQ ID NO: 82, and the amino acid sequence of the light chain is SEQ ID NO: 83; or
the amino acid sequence of the heavy chain is SEQ ID NO: 84, and the amino acid sequence of the light chain is SEQ ID NO: 85; or
the amino acid sequence of the heavy chain is SEQ ID NO: 86, and the amino acid sequence of the light chain is SEQ ID NO: 87; or
the amino acid sequence of the heavy chain is SEQ ID NO: 88, and the amino acid sequence of the light chain is SEQ ID NO: 89; or
the amino acid sequence of the heavy chain is SEQ ID NO: 90, and the amino acid sequence of the light chain is SEQ ID NO: 91.

9. Use of the antibody or antigen-binding fragment thereof according to any one of claims 1 to 8 in the manufacture of a medicament for activating PD-1 activity or PD-1 expression level, enhancing immune suppression by PD-1 in a subject, inhibiting T lymphocytes, inhibiting the expression of cytokines such as IFN-γ by T lymphocytes, and for the prevention, treatment or adjuvant treatment of inflammation and autoimmune diseases.

10. A nucleic acid molecule encoding the antibody or antigen-binding fragment thereof according to any one of claims 1 to 9.

11. An expression vector, wherein the vector comprises the sequence of the nucleic acid molecule according to claim 10 and expression regulatory sequences associated with the sequence.

12. A monoclonal antibody conjugate, comprising a monoclonal antibody and a conjugate moiety, wherein the monoclonal antibody is the antibody or antigen-binding fragment thereof according to any one of claims 1 to 8, and the conjugate moiety is selected from one or more of radionuclides, agents, toxins, cytokines, cytokine receptor fragments, enzymes, fluorophores and biotin.

13. Use of the monoclonal antibody conjugate according to claim 12 in the manufacture of a medicament for activating PD-1 activity or PD-1 expression level, enhancing immune suppression by PD-1 in a subject, inhibiting T lymphocytes, inhibiting the expression of cytokines by T lymphocytes, and preventing or treating inflammation, cancers and autoimmune diseases.
